(19) **Europäisches Patentamt**
**European Patent Office**
**Office européen des brevets**

(11) **EP 4 570 311 A1**

(12) **EUROPEAN PATENT APPLICATION**
published in accordance with Art. 153(4) EPC

(43) Date of publication:
**18.06.2025 Bulletin 2025/25**

(21) Application number: **23852223.9**

(22) Date of filing: **02.06.2023**

(51) International Patent Classification (IPC):
***A61N 5/10*** (2006.01)

(52) Cooperative Patent Classification (CPC):
**A61N 5/10**

(86) International application number:
**PCT/JP2023/020710**

(87) International publication number:
**WO 2024/034239 (15.02.2024 Gazette 2024/07)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.08.2022 JP 2022126627**

(71) Applicant: **B dot Medical Inc.**
**Tokyo 134-0003 (JP)**

(72) Inventors:
• **HARA Yousuke**
**Tokyo 134-0003 (JP)**
• **KUBOTA Yoshiki**
**Tokyo 134-0003 (JP)**
• **MINOHARA Shinichi**
**Tokyo 134-0003 (JP)**

(74) Representative: **AWA Sweden AB**
**Matrosgatan 1**
**Box 5117**
**200 71 Malmö (SE)**

(54) **CHARGED PARTICLE BEAM IRRADIATION SYSTEM**

(57) There is provided a charged particle beam irradiation system capable of realizing respiratory gated irradiation using X-rays in a charged particle beam irradiation apparatus that is not of a rotary gantry type. A charged particle beam irradiation system includes a charged particle beam irradiation apparatus on which a charged particle beam transported after being emitted from an accelerator is incident and from which the charged particle beam can be emitted toward an isocenter; and a first X-ray generation unit and a first detection unit; and a second X-ray generation unit and a second detection unit, respective X-rays generated from the first X-ray generation unit and the second X-ray generation unit pass through the isocenter and are detected by the first detection unit and the second detection unit, the first X-ray generation unit and the second X-ray generation unit are disposed to sandwich a virtual plane therebetween, the virtual plane being formed by a plurality of trajectories of the charged particle beam selectable by the charged particle beam irradiation apparatus, and when a side where the charged particle beam is incident on the charged particle beam irradiation apparatus is defined as an upstream side and a side where the charged particle beam is emitted from the charged particle beam irradiation apparatus is defined as a downstream side, the first detection unit and the second detection unit are located upstream or downstream of the first X-ray generation unit and the second X-ray generation unit.

FIG. 9

## Description

Technical Field

The present invention relates to the charged particle beam irradiation system.

Background Art

In the related art, particle beam treatment (sometimes called proton beam treatment) for irradiating an affected area with a charged particle beam such as a proton beam or a heavy particle beam (for example, a carbon beam) to perform treatment has been performed as a cancer treatment method. In such particle beam treatment, the dose of the charged particle beam to the affected area is increased by irradiating the affected area with the charged particle beam from various directions while concentrating the charged particle beam on the affected area to suppress exposure of areas other than the affected area. Patent Literature 1 and 2 disclose an apparatus that radiates a charged particle beam, which is a rotating irradiation apparatus, in which a beam transport system and an irradiation unit are configured to rotate around a patient so that the patient can be irradiated with the charged particle beam from all directions. However, such a rotating irradiation apparatus (hereinafter referred to as a rotary gantry) becomes large because the rotating irradiation apparatus is configured to rotate around the patient. Therefore, Patent Literature 3 discloses an apparatus that radiates a charged particle beam, which is a charged particle beam irradiation apparatus that can radiate a charged particle beam from any angle without using a rotating irradiation apparatus. The charged particle beam irradiation apparatus described in Patent Literature 3 can be made smaller than those in Patent Literature 1 and 2 by not using a mechanism for rotating the irradiation unit, which is one of causes of the apparatus becoming larger.

Citation List

Patent Literature

Patent Literature 1: JP 6523076 B
Patent Literature 2: JP 6158334 B
Patent Literature 3: JP 6387476 B

Summary of Invention

Technical Problem

Incidentally, the charged particle beam must be radiated accurately and precisely to a patient's treatment site. However, since a patient's organs are constantly moving due to the patient's breathing, pulsation, or the like, there is a problem that the treatment site is constantly moving at an irradiation position of the charged particle beam. Therefore, there is an irradiation method called respiratory gated irradiation, which is a treatment that is performed in consideration of patient's breathing, or the like. An example of realizing this irradiation method includes a method of monitoring a state of a tumor, markers placed around the tumor, or organs inside a patient with X-rays at the time of treatment irradiation to confirm a treatment site as an irradiation position of a charged particle beam, and controlling the radiation of the charged particle beam at appropriate timing. An X-ray generation unit and a detection unit that perform the monitoring need to be installed in suitable positions where high-precision detection can be realized, but in the case of a rotary gantry such as those in Patent Literature 1 and 2, there are restrictions on installation spaces, and the X-ray generation unit and the detection unit often need to be installed in specific positions. For example, an X-ray generation unit and a detection unit facing the X-ray generation unit are provided so that the X-ray generation unit and the detection unit rotate coaxially like the charged particle beam irradiation system and do not interfere with the radiation of the charged particle beam, making it possible to detect the state of the organs inside the patient and radiate the charged particle beam. When a rotary gantry with a rotation angle of 360 degrees or more (hereinafter, full gantry) as disclosed in Patent Literature 1 is used, the full gantry is generally configured so that a plane formed by an irradiation axis of each charged particle beam generated at the time of irradiation from different angles and a plane formed by an axis connecting the X-ray generation unit to the detection unit are formed on the same plane. In other words, the X-ray generation unit and the detection unit also rotate as the irradiation unit rotates. On the other hand, in the case of a rotary gantry with an angle of less than 360 degrees (for example, 180 degrees) as disclosed in Patent Literature 2 (hereinafter, half gantry), an X-ray generation unit and a detection unit may be installed while not rotating at the same time as an irradiation unit, unlike a full gantry. The installation accuracy of the X-ray generation unit and the detection unit, and the repeatability of positions of the X-ray generation unit and the detection unit after rotation are improved due to not rotating. On the other hand, in the case of a charged particle beam irradiation apparatus different from a rotary gantry such as that described in Patent Literature 3, it is difficult to dispose the plane formed by the irradiation axis of the charged particle beam and the plane formed by the axis connecting the X-ray generation unit to the detection unit on the same plane. For example, when equipment is disposed so that X-rays and proton beams travel along a cross section perpendicular to a patient's craniocaudal direction, there is a possibility that an irradiation nozzle and a flat panel detector (FPD) which is a detection unit may interfere with each other, or the X-ray generation unit on a floor surface may interfere with an irradiation apparatus, making realization difficult.

The present invention has been made in consideration of the above problems, and an object of the present invention is to provide a charged particle beam irradiation system capable of treatment using respiratory gated irradiation with X-rays even in the case of a non-rotary gantry.

Solution to Problem

To solve the above problem, a charged particle beam irradiation system according to an aspect of the present invention includes a charged particle beam irradiation apparatus on which a charged particle beam transported after being emitted from an accelerator is incident and from which the charged particle beam is emitted toward an isocenter; and a first X-ray generation unit and a first detection unit, and a second X-ray generation unit and a second detection unit, wherein respective X-rays generated from the first X-ray generation unit and the second X-ray generation unit pass through the isocenter and are detected by the first detection unit and the second detection unit, the first X-ray generation unit and the second X-ray generation unit are disposed to sandwich a virtual plane between the first X-ray generation unit and the second X-ray generation unit, the virtual plane being formed by a plurality of trajectories of the charged particle beam selectable by the charged particle beam irradiation apparatus, and when a side where the charged particle beam is incident on the charged particle beam irradiation apparatus is defined as an upstream side and a side where the charged particle beam is emitted from the charged particle beam irradiation apparatus is defined as a downstream side, the first detection unit and the second detection unit are located upstream or downstream of the first X-ray generation unit and the second X-ray generation unit.

According to the charged particle beam irradiation system according to the present invention, treatment using respiratory gated irradiation with X-rays can be performed even when a gantry is not a rotary gantry.

Brief Description of Drawings

FIG. 1 is a schematic diagram of a particle beam treatment facility.

FIG. 2(a) is a side view of the vicinity of an irradiation nozzle, and FIG. 2(b) is a front view of the vicinity of the irradiation nozzle.

FIG. 3 is a perspective view of a charged particle beam irradiation system in a state in which an X-ray generation unit is not disposed.

FIG. 4(a) is a left side view of the charged particle beam irradiation system illustrated in FIG. 3, and FIG. 4(b) is a right side view of the same charged particle beam irradiation system.

FIG. 5 is a front view of the charged particle beam irradiation system illustrated in FIG. 3.

FIG. 6(a) is a top view of the charged particle beam irradiation system illustrated in FIG. 3, and FIG. 6(b) is a rear view of the charged particle beam irradiation system.

FIG. 7 is a perspective view of the charged particle beam irradiation system in which an X-ray generation unit, a detection unit thereof, and a movable vehicle on which a patient is placed are disposed.

FIG. 8 is a right side view of the charged particle beam irradiation system illustrated in FIG. 7.

FIG. 9 is a front view of the charged particle beam irradiation system illustrated in FIG. 7.

FIG. 10 is a view illustrating a mechanism of radiation of a charged particle beam in a charged particle beam irradiation apparatus.

FIG. 11 is an example of a top view of a treatment room including the charged particle beam irradiation apparatus.

FIG. 12 is a schematic view illustrating installation conditions of an X-ray imaging apparatus.

FIG. 13 is a diagram illustrating a relationship between an effective field of view and an installation angle of the X-ray imaging apparatus.

FIG. 14 is a system configuration diagram illustrating a system configuration example of the charged particle beam irradiation apparatus.

FIG. 15 is a block diagram illustrating a configuration example of an information processing device that controls radiation of a charged particle beam in the charged particle beam irradiation apparatus.

FIG. 16 is a flowchart illustrating an example of an operation of controlling the charged particle beam irradiation apparatus in the information processing device.

FIG. 17 is an example of a timing chart when respiratory waveform prediction is performed and treatment irradiation is performed.

FIG. 18 is another example of a timing chart when the respiratory waveform prediction is performed and the treatment irradiation is performed.

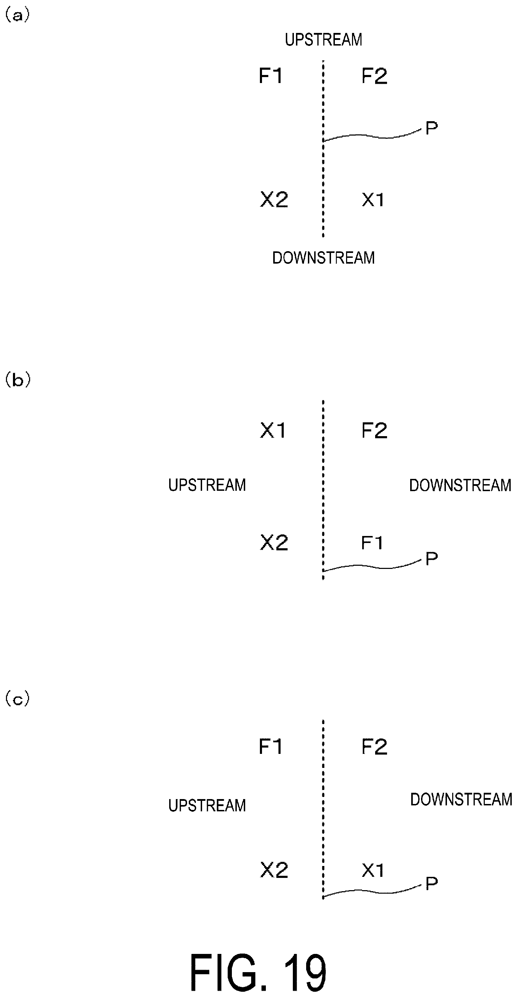

FIG. 19(a) is a diagram illustrating a disposition relationship among the charged particle beam irradiation system, the X-ray generation unit, and the detection unit according to the present embodiment, FIG. 19(b) is a diagram illustrating a disposition relationship among a charged particle beam irradiation system, an X-ray generation unit, and a detection unit in a full gantry of the related art, and FIG. 19(c) is a diagram illustrating a disposition relationship among a charged particle beam irradiation system, an X-ray arrangement unit, and a detection unit in a half gantry of the related art.

Description of Embodiments

Hereinafter, a charged particle beam irradiation system according to the present embodiment will be described in detail with reference to the drawings.

Embodiments

Example 1

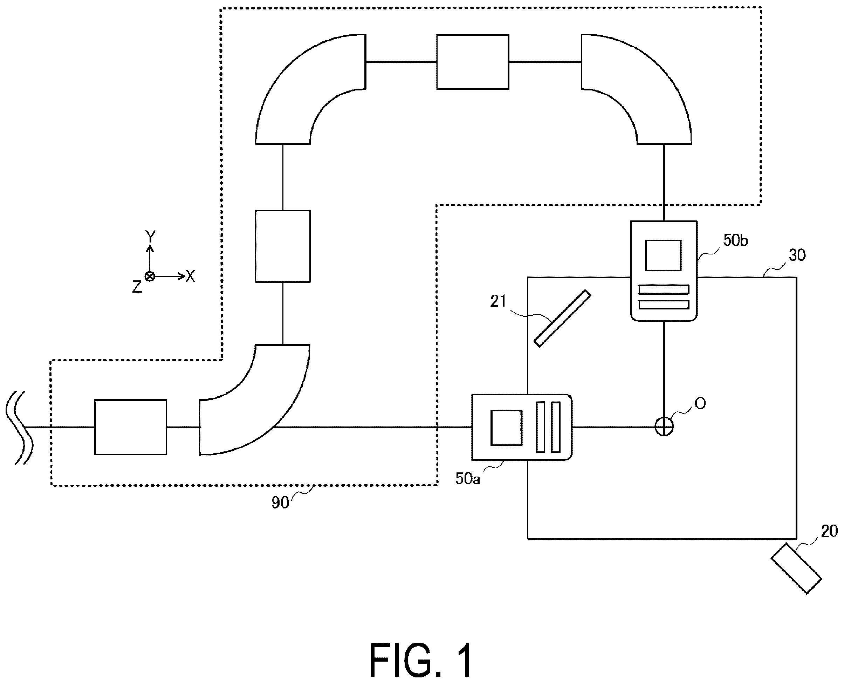

FIG. 1 is a schematic diagram of a particle beam treatment facility in which the present invention is carried out. A charged particle beam extracted from an accelerator (not illustrated) passes through a beam transport system 90 and is transported to a treatment room 30. Here, the accelerator is an apparatus that generates a charged particle beam, and is realized by, for example, a synchrotron, a cyclotron, or a linear accelerator. The beam transport system 90 includes a vacuum duct and a charged particle beam adjustment apparatus. The charged particle beam adjustment apparatus includes a beam slit for adjusting a beam shape and/or dose, a deflection electromagnet for adjusting a traveling direction of a charged particle beam, a quadrupole electromagnet for adjusting a beam shape of the charged particle beam, and a steering electromagnet for finely adjusting a beam position of the charged particle beam, appropriately according to a specification, and adjusts a beam shape and dose of the charged particle beam. All four surrounding sides of the treatment room are walls made of concrete in consideration of radiation shielding. The treatment room at the end of the beam transport system has an irradiation nozzle, and the charged particle beam passes through the irradiation nozzle and is radiated to a patient lying on a treatment table. FIG. 1 discloses an example in which irradiation can be performed from two directions, horizontally (irradiation nozzle of a charged particle beam irradiation apparatus 50a) and vertically (irradiation nozzle of a charged particle beam irradiation apparatus 50b), in the treatment room. In Example 1, a horizontal traveling direction of the charged particle beam is defined as X, a vertical traveling direction is defined as Y, and a direction perpendicular to both X and Y is defined as Z. The charged particle beams traveling in the horizontal and vertical directions intersect at a point O called an isocenter. The irradiation nozzle includes a scanning electromagnet for performing scanning with the charged particle beam in an irradiation target shape, a dose monitor for measuring a dose, a position monitor for measuring a beam position, an energy modulation device, and the like. Further, the treatment room includes a positioning apparatus for performing patient positioning. Patient positioning may refer to specifying a relative positional relationship of the patient (treatment site) with respect to the irradiation nozzle (with respect to the charged particle beam irradiation apparatus) in the treatment room. The positioning apparatus includes an image diagnostic apparatus configured of an X-ray tube as an X-ray generation unit 20 and a flat panel detector (hereinafter, FPD) as the detection unit, and a device for transmitting and receiving positioning data. In FIG. 1, two FPDs 21 (only one in the figure) are installed to be suspended from a ceiling (not illustrated). The X-ray tube 20 is installed at a position symmetrical to the FPD 21 with respect to the point O. In FIG. 1, two X-ray tubes are installed under a floor. In the present Example 1, the FPD 21 is on the ceiling side and the X-ray tube 20 is under the floor, but this does not limit the disposition of the FPD 21 and the X-ray tube 20, and the FPD may be on the floor side and the X-ray tube may be on the ceiling side. Although not illustrated, other image diagnostic apparatuses, such as an X-ray CT or MRI apparatus may be installed in the treatment room. Further, in the present example, the FPD 21 is suspended from the ceiling, but the present invention is not limited thereto, and the FPD may be attached to an apparatus in the treatment room.

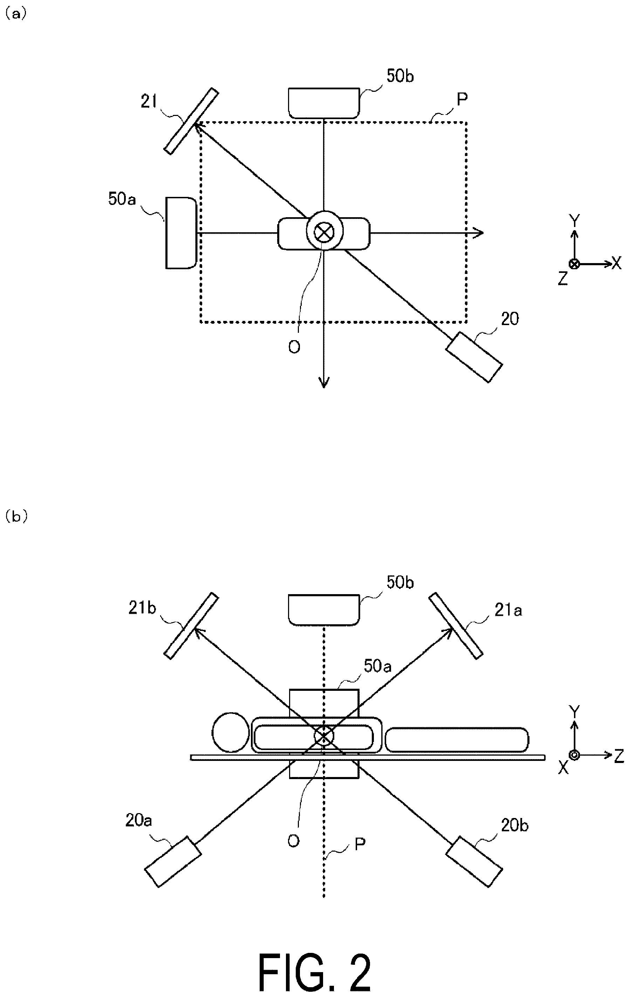

FIG. 2(a) is a schematic diagram of a side view of the vicinity of the irradiation nozzle that realizes Example 1, and FIG. 2(b) is a front view of the vicinity of the irradiation nozzle that realizes Example 1. In FIG. 2, a virtual plane formed by the charged particle beams in two directions including a horizontal direction and a vertical direction is defined as a virtual plane P, and the point O is within the virtual plane P. When pairs of positioning apparatuses are an X-ray tube 20a and an FPD 21a, and an X-ray tube 20b and an FPD 21b, the FPD 21a and the FPD 21b are installed upstream of the X-ray tube 20a and the X-ray tube 20b in a traveling direction of the charged particle beam, as illustrated in FIG. 2(a). In the present example, the FPD 21 is installed upstream of the X-ray tube 20, but the FPD 21 may be installed downstream of the X-ray tube 20. The X-ray tube 20a and the X-ray tube 20b are located symmetrically with respect to the virtual plane P. The FPD 21a and the FPD 21b are located symmetrically with respect to the virtual plane P. Since the X-ray tube 20a and the FPD 21a, and the X-ray tube 20b and the FPD 21b are installed symmetrically with respect to the point O as illustrated in FIG. 2(b), the X-ray tube 20a and the FPD 21b are disposed on the same side of the virtual plane P, and the X-ray tube 20b and the FPD 21a are disposed on the opposite side. Although an example of disposition symmetrically with respect to the virtual plane P is illustrated here, the X-ray tube 20a and the X-ray tube 20b may not be disposed symmetrically with respect to the virtual plane P, and for example, the FPD 21a may be disposed further back (further behind the plane of the paper) than illustrated in the drawing in a front-back (depth) direction of the paper in FIG. 2(b).

As illustrated in FIG. 2, a Z direction is the patient's craniocaudal direction. Although not illustrated, the patient lies on the treatment table, and patient positioning and treatment irradiation are performed. In the present example, since the FPD 21a and the FPD 21b, and the X-ray tube 20a and the X-ray tube 20b are installed to sandwich the virtual plane P therebetween, a wide area in the craniocaudal direction can be imaged. When the X-ray tube 20a and the X-ray tube 20b, and the FPD 21a and the FPD 21b are disposed on the same side with respect to the virtual plane P, that is, when the X-ray tube 20a and the X-ray tube 20b, and the FPD 21a and the FPD 21b are not disposed symmetrically with respect to the virtual plane P, left and right sides of a body axis are imaging areas, and a human body cannot be imaged over a wide area. As a result, the patient is moved in the craniocaudal direction and imaged multiple times in order to image a wide range. In the present example, since a wide range can be imaged at once, an amount of radiation exposure due to X-ray imaging can be reduced. Further, a positioning work time is shortened to improve utilization efficiency of the treatment room, thereby contributing to an increase in hospital's profits.

In the present Example 1, as illustrated in FIG. 2(a), the two FPDs 21 (21a and 21b) can be installed between a horizontal irradiation nozzle and a vertical irradiation nozzle, and the space can be effectively used. In general, in order to make it easier for a radiographer to ascertain a patient's posture at a three-dimensional position when positioning the patient, the X-ray tube 20 and the FPD 21 are installed so that the virtual plane P formed by an irradiation axis of the charged particle beam and a plane formed by an axis connecting the X-ray tube 20 to the FPD 21 are on the same plane. Alternatively, the X-ray tube 20 and the FPD 21 are installed so that the plane formed by the axis connecting the X-ray tube 20 to the FPD 21 is inclined with respect to the horizontal side relative to the virtual plane P. That is, one of the FPDs 21 is installed downstream in the beam travel direction on the open space side of the treatment room. In this case, it becomes difficult to access an irradiation port from the downstream side in a horizontal beam travel direction, and a configuration such as retracting the FPD 21 is required to prevent interference between the FPD 21 and a person. In the present example, it is possible to access the upstream side from the downstream side in the horizontal beam travel direction without retracting the FPD 21, and work efficiency can be improved. It is expected that the installation position accuracy will be improved by eliminating the need to retract the FPD 21, and high-precision treatment irradiation such as respiratory gated irradiation is possible. Further, when the FPD 21 cannot be installed upstream, the treatment table may overlap an imaging range, and there is concern that imaging conditions may change. In the present example, this situation can be avoided, the imaging conditions can be made uniform regardless of a position of the treatment table, and it is expected that the accuracy of treatment will be improved.

Example 2

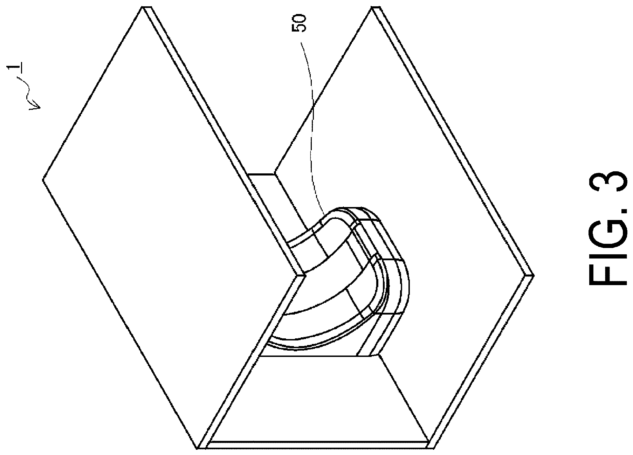

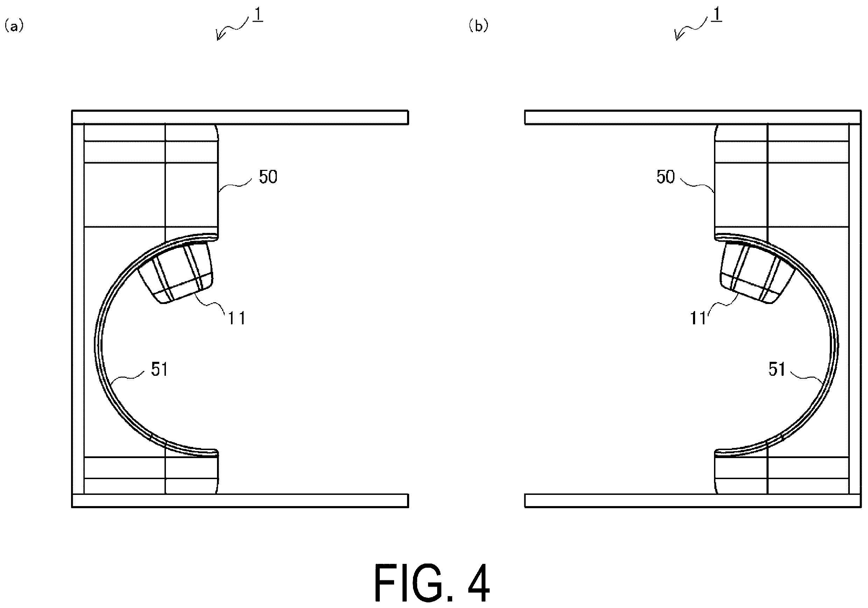

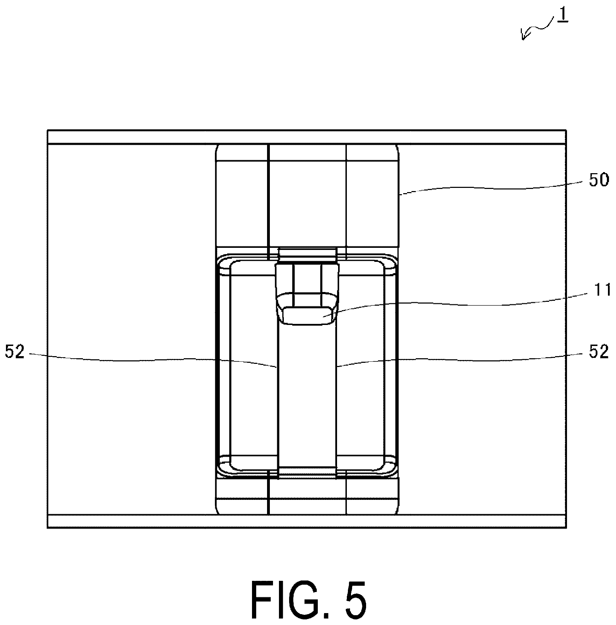

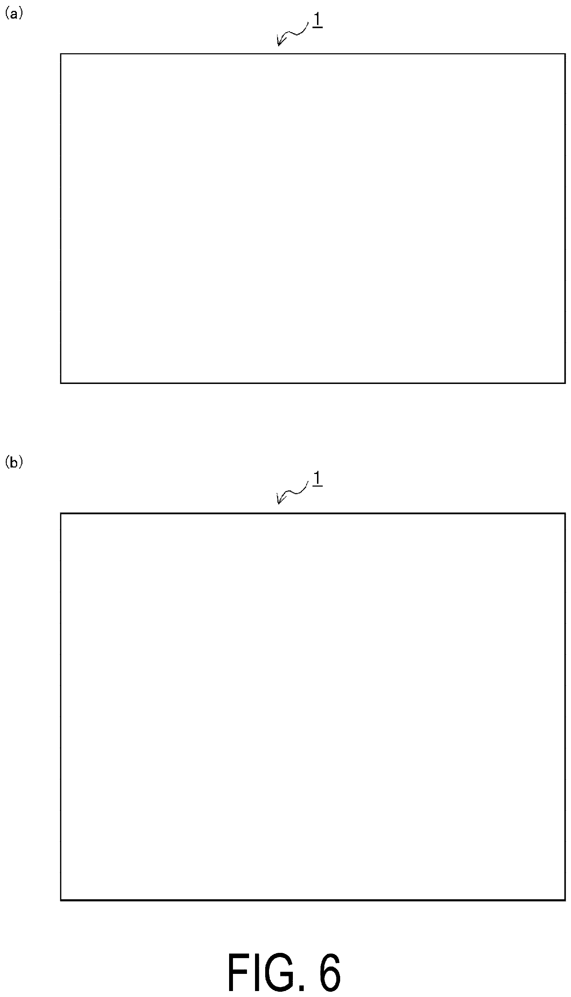

FIG. 3 is a perspective view of the charged particle beam irradiation system in a state in which a charged particle beam irradiation apparatus in a state in which an X-ray generation unit and a detection apparatus for detecting X-rays are not installed is not installed. FIG. 4(a) is a right side view of the charged particle beam irradiation apparatus illustrated in FIG. 3. FIG. 4(b) is a left side view of the charged particle beam irradiation apparatus. FIG. 5 is a front view of the charged particle beam irradiation apparatus illustrated in FIG. 3. FIG. 6(a) is a top view of the charged particle beam irradiation apparatus illustrated in FIG. 3, and FIG. 6(b) is a rear view of the charged particle beam irradiation apparatus.

As illustrated in FIG. 3 to FIG. 6 (particularly FIG. 4), the charged particle beam irradiation apparatus according to Example 2 has a shape with a partial semicircular cutout when viewed from the side, and a charged particle beam is radiated from a semicircular recess 51 toward the isocenter O at a center of a semicircle. An irradiation nozzle 11 is provided in the recess 51, and the charged particle beam is radiated from the irradiation nozzle 11 to the affected area (isocenter) that is the irradiation target. The irradiation nozzle 11 can slide within a semicircular range along a guide rail 52 provided in the recess 51 of the charged particle beam irradiation apparatus, and radiates the charged particle beam from various directions within this range. The irradiation nozzle 11 is not an essential component, and the charged particle beam can be radiated from a concave surface of the recess 51 even without the irradiation nozzle 11.

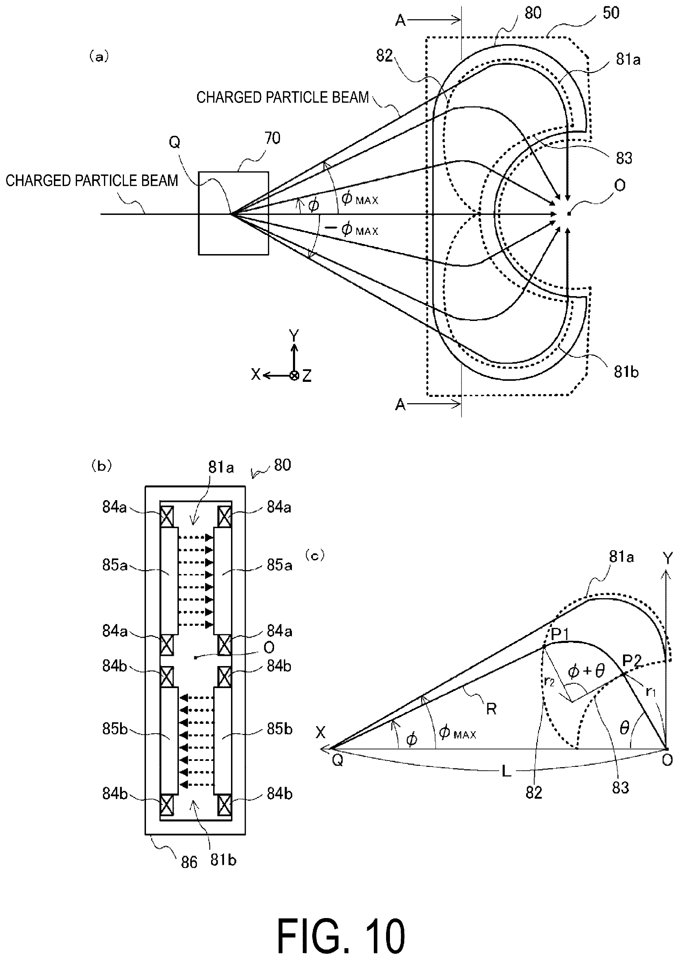

Here, a mechanism of radiation of the charged particle beam by the charged particle beam irradiation apparatus (non-rotary gantry) in the present Example 2 will be briefly described using FIG. 10. The irradiation nozzle 11 is omitted in FIG. 10.

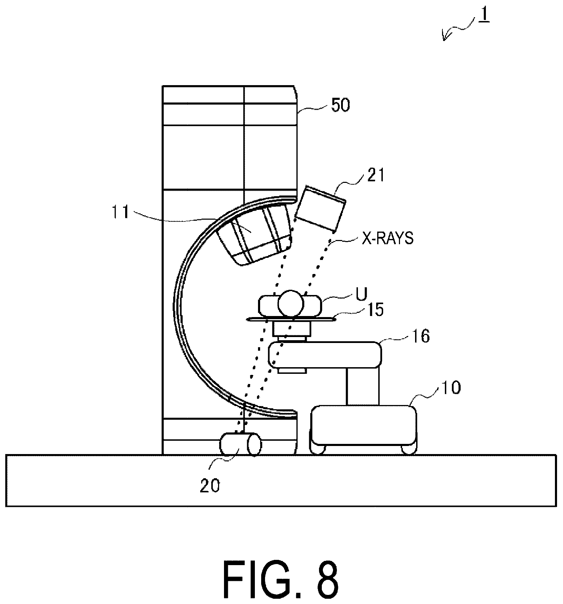

FIG. 10(a) is a schematic diagram schematically illustrating paths of the charged particle beam as viewed from the right side of a deflection electromagnet 80 provided in a charged particle beam irradiation apparatus 50 of the charged particle beam irradiation system. That is, FIG. 10(a) corresponds to FIG. 4(a) or FIG. 8 which will be described later. As illustrated in FIG. 10(a), the charged particle beam irradiation apparatus 50 includes a distribution electromagnet 70 and the deflection electromagnet 80.

The charged particle beam (left end of FIG. 10) input to the charged particle beam irradiation apparatus is accelerated by an accelerator (not illustrated) and input to the charged particle beam irradiation apparatus via a beam transport system (not illustrated). For further details of the charged particle beam irradiation apparatus, please refer to Patent Literature 3.

FIG. 10(a) illustrates an example of a plurality of beam paths that differ for each deflection angle $\varphi$ and convergence angle $\theta$. Here, a traveling direction of the charged particle beam is defined as an X-axis, a direction of a magnetic field generated by the deflection electromagnet 80 is defined as a Z-axis, and a direction perpendicular to the X-axis and Z-axis is defined as a Y-axis. The deflection electromagnet 80 is configured to converge the charged particle beam incident from a wide range of deflection angles $\varphi$ with respect to the X-axis on an XY plane on the isocenter O. In FIG. 10(a), the irradiation nozzle is omitted, and for simplicity of the description, the isocenter O is set as an origin of an XYZ space, and the upstream side (the accelerator side and the left side of the paper in FIG. 10(a)) is set as a positive direction of the X axis.

A range of the deflection angle $\varphi$ is greater than -90 degrees and less than +90 degrees, and a positive (+Y-axis) deflection angle range and a negative (-Y-axis) deflection angle range may be different (asymmetric). For example, a maximum deflection angle on the positive side ($\varphi = \varphi$MAX) may be any of 10 degrees, 15 degrees, 20 degrees, 25 degrees, 30 degrees, 35 degrees, 40 degrees, 45 degrees, 50 degrees, 60 degrees, 70 degrees, 80 degrees, and 85 degrees, and a maximum deflection angle on the negative side ($\varphi = -\varphi$MAX) may be any of -10 degrees, -15 degrees, -20 degrees, -25 degrees, -30 degrees, -35 degrees, -40 degrees, -45 degrees, -50 degrees, -60 degrees, -70 degrees, -80 degrees, and -85 degrees. The deflection angle $\varphi$ is not limited to these angles.

The deflection electromagnet 80 includes one or more pairs of coils, and the pair of coils are disposed to generate a uniform magnetic field (effective magnetic field regions 81a and 81b) that is oriented in a direction perpendicular to the traveling direction of the charged particle beam and a direction of spread of the deflection angle $\varphi$ of the charged particle beam (Z-axis direction in the figure), and to sandwich the path of the charged particle beam therebetween. The effective magnetic field region generated by one pair of coils of the deflection electromagnet 80 has a crescent shape on the XY plane as illustrated in FIG. 10(a), and details thereof will be described later. Since a gap between the pair of opposing coils through which the charged particle beam passes (distance in the Z-axis direction) is sufficiently small compared to a range in which the charged particle beam spreads on the XY plane, the spread of the charged particle beam in the Z-axis direction is not taken into consideration here.

FIG. 10(b) is a cross-sectional view of the deflection electromagnet 80 taken along line A-A. The deflection electromagnet 80 preferably includes at least two pairs of coils 84a and 84b. Magnetic poles 85a and 85b are built into the coils 84a and 84b, respectively, and a yoke 86 is connected to the magnetic poles 85a and 85b. When a power supply (not illustrated) is connected to the deflection electromagnet 80, and when a current (excitation current) is supplied from the power supply to the pairs of coils 84a and 84b, the deflection electromagnet 80 is excited and the effective magnetic field regions 81a and 81b (collectively referred to as effective magnetic field region 81) are formed.

A range of the effective magnetic field region 81a and a range of the effective magnetic field region 81b may be different (asymmetric). For example, when a range of the positive (+Y-axis) deflection angle $\varphi$ and a range of the negative (-Y-axis) deflection angle $\varphi$ are asymmetric, the effective magnetic field regions 81a and 81b are formed asymmetrically accordingly, thereby making it possible to reduce the unused effective magnetic field region.

A range of the deflection angle $\varphi$ of the charged particle beam deflected by the distribution electromagnet 70 and incident on the deflection electromagnet 80 is a range from a positive maximum deflection angle ($\varphi = \varphi$MAX) to a negative maximum deflection angle ($\varphi = -\varphi$MAX), the positive maximum deflection angle $\varphi$MAX is an angle of 10 degrees or more and less than 90 degrees, and the negative maximum deflection angle $-\varphi$MAX is an angle of greater than -90 degrees and -10 degrees or less. The deflection angle $\varphi$ and an irradiation angle $\theta$ to be described later are angles of paths of the charged particle beam relative to the X-axis on the XY plane.

The charged particle beam incident in the positive deflection angle range ($\varphi$ is from more than 0 to $\varphi$MAX) is deflected by the effective magnetic field region 81a of the first pair of coils 84a, passes through the irradiation nozzle 11, and is radiated to the isocenter O. The charged particle beam incident in the negative deflection angle range ($\varphi$ is from less than 0 to $-\varphi$MAX) is deflected by the effective magnetic field region 81b of the second pair of coils 84b, passes through the irradiation nozzle 11, and is radiated to the isocenter O. The magnetic fields of the effective magnetic field regions 81a and 81b are oriented in opposite directions. The charged particle beam incident on the deflection electromagnet 80 at the deflection angle $\varphi$ of 0 from the distribution electromagnet 70 passes through either or both of the effective magnetic field regions 81a and 81b, and is converged on the isocenter O through the irradiation nozzle (not illustrated).

The deflection angle $\varphi$ of the charged particle beam incident on the deflection electromagnet 80 is controlled by the distribution electromagnet 70. The distribution electromagnet 70 includes an electromagnet that generates a magnetic field oriented in a direction (Z-axis in the figure) perpendicular to a traveling direction (X-axis in the figure) of the charged particle beam supplied from the accelerator (not illustrated), and deflects the passing charged particle beam, and a control unit that controls a strength and direction of the magnetic field (both not illustrated). The distribution electromagnet 70 controls the strength and direction (Z-axis direction) of the magnetic field to deflect the charged particle beam on the XY plane, and emits the charged particle beam deflected at the deflection angle φ at a deflection starting point Q to the deflection electromagnet 80. Here, the deflection starting point Q and the isocenter O are on the X-axis (on the same horizontal plane).

A calculation equation for forming the effective magnetic field region 81a of the deflection electromagnet 80 will be described with reference to FIG. 10(c). In the present embodiment, the formation of the effective magnetic field region on the XY plane will be described since the deflection of the charged particle beam in the Z-axis direction is not taken into consideration. The effective magnetic field region 81a of the deflection electromagnet 80 will be described, and since the same applies to the effective magnetic field region 81b, description of the effective magnetic field region 81b will be omitted.

First, the boundary of the effective magnetic field region 81a on an emission side 83 of the charged particle beam in the deflection electromagnet 80 is determined to be in a range at an equal distance r1 from the isocenter O. Next, the boundary of the effective magnetic field region 81a on an incidence side 82 of the charged particle beam in the deflection electromagnet 80 is determined based on relational Equations (1) to (5) to be described later so that the incident charged particle beam deflected at the deflection angle φ at the imaginary deflection starting point Q located at a predetermined distance L from the isocenter O converges on the isocenter O. Here, the imaginary deflection starting point Q is a point at which the charged particle beam is assumed to receive a kick of the deflection angle φ over an extremely short distance at a center of the distribution electromagnet 70.

The charged particle beam transported at the deflection angle φ enters from any point P1 on the boundary of the effective magnetic field region 81a on the incidence side 82, performs a circular motion with a curvature radius r2 within the effective magnetic field region 81a (a central angle in this case is ($\varphi + \theta$)), exits from a point P2 on the boundary of the effective magnetic field region 81a on the emission side 83, and is radiated toward the isocenter O. In other words, the points P1 and P2 are on an arc with the radius r2 and the central angle ($\varphi + \theta$).

As illustrated in FIG. 10(c), an XY coordinate system with the isocenter O as an origin on the XY plane is assumed. When an angle between a line connecting the point P2 on the emission side 83 to the isocenter O and the X axis is the irradiation angle θ, coordinates (x, y) of the point P1 on the incidence side 82, the deflection angle φ, and a distance R between the point Q and the point P1 can be calculated from the following relational Equations (1) to (4).

[Math. 1]

$$x = r_1 \cos\theta + r_2(\sin\theta + \sin\varPhi) \quad (1)$$
$$y = r_1 \sin\theta - r_2(\cos\theta - \cos\varPhi) \quad (2)$$

$$\varPhi = \sin^{-1}\left(\frac{r_2}{\sqrt{R^2 + r_2^2}}\right) + \sin^{-1}\left(\frac{r_1 \sin\theta - r_2 \cos\theta}{\sqrt{R^2 + r_2^2}}\right) \quad (3)$$

$$R = \sqrt{L^2 + r_1^2 - 2L(r_1 \cos\theta + r_2 \sin\theta)} \quad (4)$$

Here, a magnetic field with a uniform magnetic flux density B is generated in the effective magnetic field region 81a, and when a momentum of the charged particle beam is p (which roughly depends on the accelerator) and a charge thereof is q, the curvature radius r2 of the charged particle beam deflected in the magnetic field is expressed by Equation (5).

[Math. 2]

$$r_2 = \frac{p}{qB} \quad (5)$$

A shape and disposition of the pair of coils 84a and the magnetic pole 85a of the deflection electromagnet 80 are adjusted based on the relational Equations (1) to (5) and a current flowing through the pair of coils 84a is adjusted so that a shape of the boundary of the effective magnetic field region 81a can be adjusted. That is, the boundary is determined so that a distance between any point P2 on the boundary of the effective magnetic field region 81a on the emission side 83 and the isocenter O becomes the equal distance r1, r2 is determined from Equation (5) by adjusting the magnetic flux density B of the effective magnetic field region 81a, and the boundary of the effective magnetic field region 81a on the incidence side 82 is determined so that the distance R between the point P1 on the boundary of the effective magnetic field region 81a on the incidence side 82 and the deflection starting point Q has a relationship of Equation (4). A maximum value of $\varphi$ in Equation (3) is the maximum deflection angle $\varphi$MAX. Although not limited thereto, disposition of the deflection starting point Q, the deflection electromagnet 80, and the isocenter O is preferably adjusted so that the charged particle beam passing through the deflection starting point Q converges on the isocenter O without being deflected by the deflection electromagnet 80 because an apparatus configuration can be further simplified.

The boundary between the effective magnetic field regions 81a and 81b of the deflection electromagnet 80 obtained as described above has an ideal shape for converging the charged particle beam on the isocenter O. In practice, even when there is a deviation from this ideal shape or non-uniformity in a magnetic field distribution, an excitation amount (the magnetic flux density B) of the deflection electromagnet 80 is finely adjusted in advance for each deflection angle $\varphi$, information about the adjustment is stored in the power supply, and the deflection angle $\varphi$ and the amount of current of the deflection electromagnet 80 are controlled to be linked to each other, thereby making it possible to deflect the charged particle beam according to the isocenter O. Further, when the non-uniformity of the magnetic field distribution can be predicted in advance, the trajectory of the charged particle beam can be finely adjusted by correcting the shape and disposition of the pairs of coils 84a and 84b and the magnetic poles 85a and 85b of the deflection electromagnet 80.

This allows the charged particle beam to be radiated to the affected area (isocenter O) at a desired angle.

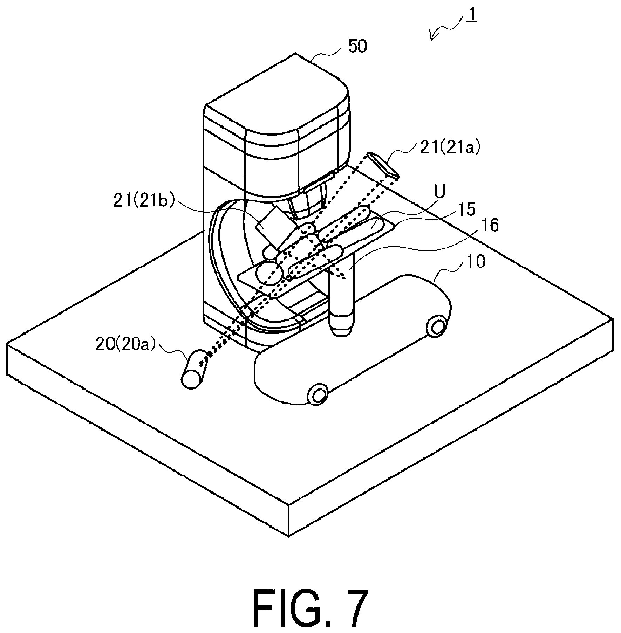

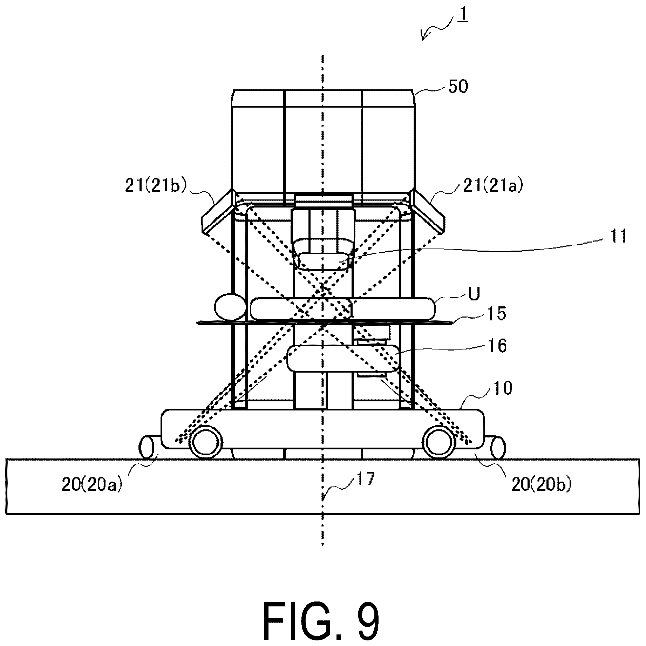

FIG. 7 is a perspective view illustrating the charged particle beam irradiation system in a state in which the X-ray generation unit 20 is disposed in the charged particle beam irradiation apparatus and a movable vehicle 10 carrying a patient is also disposed. FIG. 8 is a right side view of the charged particle beam irradiation apparatus illustrated in FIG. 7. FIG. 9 is a front view of the charged particle beam irradiation system illustrated in FIG. 3. A positional relationship between the charged particle beam irradiation apparatus, the X-ray generation unit, and detection unit thereof will be described with reference to FIGS. 7 to 9. In FIGS. 7 to 9, in order to make it easier to see the movable vehicle 10 with the X-ray generation unit irradiation apparatus or the patient, a structure on the accelerator side beyond a wall of a top surface, a wall of a back surface, and a wall of the charged particle beam irradiation apparatus 50 illustrated in FIG. 3 is not illustrated.

The charged particle beam irradiation apparatus is installed in a predetermined treatment room. A patient U who is a treatment target is placed on a treatment table 15 of the movable vehicle 10 and is transported to a treatment position of the charged particle beam irradiation apparatus by automatic control. The treatment table 15 on which the patient U is placed is connected to an arm 16 provided on the movable vehicle 10, and a treatment site of the patient U placed on the treatment table 15 can be moved to a position of the isocenter O of the charged particle beam irradiation apparatus by driving the arm 16 (rotating an axis of the arm 16). The movable vehicle 10 may be automatically controlled by a program or manually controlled by an operator using remote control.

As illustrated in FIGS. 7 to 9, the charged particle beam irradiation system 1 includes the charged particle beam irradiation apparatus 50, the X-ray generation unit 20 (20a and 20b), and a detection unit 21 (21a and 21b). As described above, the charged particle beam irradiation apparatus 50 is an apparatus that can irradiate the isocenter with a charged particle beam.

The X-ray generation unit 20 is an apparatus that radiates X-rays, and the detection unit 21 is an apparatus that faces the X-ray generation unit 20 and detects X-rays that have passed through a body of the patient U to generate an X-ray image. In FIGS. 7 to 9, the X-rays are illustrated diagrammatically by dotted lines. The X-ray image generated by the detection unit 21 may be a moving image or a still image. The X-ray image generated by the detection unit 21 is transmitted to an information processing device 100 (described later) that determines timing of radiating the charged particle beam.

As illustrated in FIGS. 7 to 9, the X-ray generation units 20 (20a and 20b) are provided in the treatment room in which the charged particle beam irradiation apparatus is disposed, near a floor surface, such as under the floor, on both sides of the charged particle beam irradiation apparatus on the irradiation nozzle 11 side (the irradiation source side of the charged particle beam) as viewed from the isocenter O. When the X-ray generation units 20 are provided under the floor, for example, it is possible to curb interfering with the movement of the vehicle 10 or the movement of people in the treatment room.

The detection units 21 (21a and 21b) that detect the X-rays irradiated from the X-ray generation units 20 are provided near a ceiling in the treatment room to face the X-ray generation units 20 (20a and 20b). That is, the detection units 21 are installed downstream of the X-ray generation units 20 in the traveling direction of the charged particle beam. Disposition positions of the X-ray generation units 20 and the detection units 21 may be reversed. That is, the detection unit 21 may be disposed upstream of the X-ray generation unit 20 in the traveling direction of the charged particle beam.

A dotted chain line (17) in FIG. 9 indicates a virtual plane 17 (corresponding to the virtual plane P described above) illustrating a path through which the charged particle beam passes. As illustrated in FIG. 9, the charged particle beam passes through the virtual plane 17, which is a vertical plane that passes through a center of the apparatus in FIG. 9. The X-rays radiated from the X-ray generation unit 20 are radiated to intersect with the virtual plane 17 and pass through the isocenter O. That is, in the charged particle beam irradiation system 1 according to the present embodiment, the X-ray generation unit 20a and the detection unit 21b are disposed on the same side not to sandwich the virtual plane 17 therebetween, and the X-ray generation unit 20b and the detection unit 21a are also disposed on the same side not to sandwich the virtual plane 17 therebetween. The X-ray generation unit 20a and the X-ray generation unit 20b may not be disposed in the treatment room to be symmetrical across the virtual plane 17, and similarly, the detection unit 21a and the detection unit 21b may not be disposed in the treatment room to be symmetrical across the virtual plane 17.

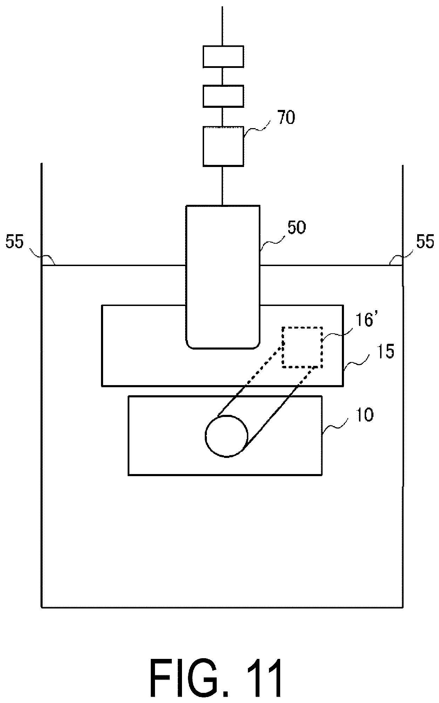

FIG. 11 is a top view of a treatment room in which the present example is realized. Dimensions of the treatment room are approximately 8 m × 6 m, but are not limited thereto. In order to ensure safety at the time of treatment and reduce the psychological burden on the patient, the charged particle beam irradiation apparatus 50 is structured so that only a part of a tip including the irradiation nozzle 11 is visible to the patient with a decorative wall 55 in between. Since the decorative wall 55 is often made of plywood, generally, when X-rays pass through the decorative wall 55, the image quality deteriorates. Therefore, the X-ray generation unit and the detection unit is preferably installed on the beam downstream side relative to the decorative wall 55. This is because, when the X-ray generation unit is installed upstream of the decorative wall 55, the X-rays will inevitably pass through the decorative wall 55 due to their positional relationship with the detection unit, which is undesirable. Therefore, the X-ray generation unit 20 and the detection unit 21 need to be installed on the beam downstream side from the decorative wall 55 illustrated in FIG. 11 so that the X-rays pass through the isocenter O. Further, when an edge of the charged particle beam irradiation apparatus 50, the irradiation nozzle 11, the arm 16 of the movable vehicle 10 and a drive mechanism 16' thereof interfere with the X-ray imaging area (broken line in FIG. 9), the X-rays are attenuated and the effective field of view is narrowed. Therefore, installation conditions that allow imaging of a wide area in the craniocaudal direction without changing the imaging conditions or enlarging the treatment room, and allow the respiratory gated irradiation will be described.

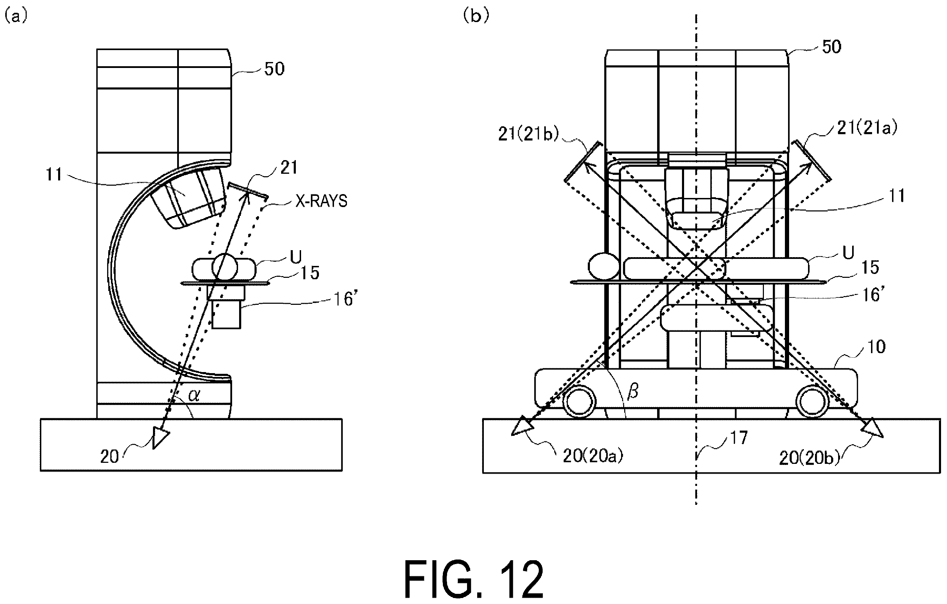

As illustrated in FIGS. 12(a) and (b), when a positional relationship between the X-ray generation unit and the detection unit when viewed from the Z direction is expressed as an angle α [deg], and a positional relationship between the X-ray generation unit and the detection unit when viewed from the X direction is expressed as an angle β [deg], an influence of α and β on the effective field of view of the detected image was confirmed. The angle α is an angle of the X-ray flux with respect to a horizontal plane on the XY plane, and the angle β is an angle of the X-ray flux with respect to the horizontal plane on a YZ plane. Here, the effective field of view refers to an area in which the X-ray flux generated from the X-ray generation unit 20 is detected by the detection unit 21 without interfering with the charged particle beam irradiation apparatus, the treatment table (movable vehicle), or the like. Further, when an entire screen area (an entire detection range of the detection unit 21) is 100 and an area occupied by the effective field of view is 80, the effective field of view is said to be 80%.

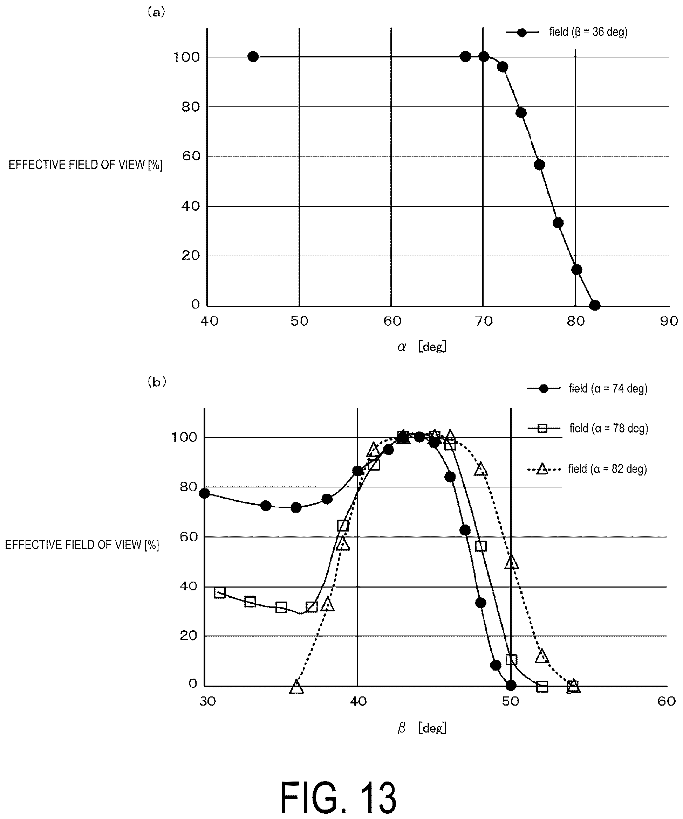

The disposition of the X-ray generation unit and the detection unit that form an ideal effective field of view is a disposition in which axes connecting two pairs of X-ray generation units 20 and detection units 21 are perpendicular to each other. As illustrated in FIG. 13(a), when β is smaller than 45 degrees, that is, the greater an opening between the axes connecting the two pairs of X-ray generation units 20 and detection units 21, the smaller the interference with the charged particle beam irradiation apparatus. On the other hand, when α is larger, that is, when an imaging system is tilted toward the irradiation apparatus, the interference between the arm 16 and the drive mechanism 16' becomes greater, and when α is smaller, that is, when the imaging system is tilted toward the floor, the interference between the arm 16 and the drive mechanism 16' becomes smaller. However, when α is smaller, the disposition of the X-ray generation unit 20 or the detection unit 21 disposed on the floor side becomes a disposition beyond the decorative wall 55 or is not within the treatment room, which is not suitable. In FIG. 13(b), the interference between the X-ray flux and a peripheral apparatus was examined when α is 70 degrees or more and β is about 45 degrees.

When α was fixed at 74 degrees and an influence of the β value on the effective field of view was confirmed, the effective field of view was 100% when $42 < β < 45$. Further, when $38 < β < 47$, the effective field of view was 80%.

When α was fixed at 78 degrees and the influence of the β value on the effective field of view was confirmed, the effective field of view was 100% when $41 < β < 46$. Further, when $39 < β < 48$, the effective field of view was 80%.

When α was fixed at 82 degrees and the influence of the β value on the effective field of view was confirmed, the effective field of view was 100% when $41 < β < 48$. Further, when $39 < β < 50$, the effective field of view was 80%.

The higher the effective field of view, the wider the imaging range of the X-ray image obtained by X-ray imaging and the better the image quality, and thus, a higher percentage of the effective field of view is better. When the X-ray generation unit and the detection unit are installed in an area in which the effective field of view is 80% or more, highly accurate treatment irradiation can be performed without increasing a size of the apparatus while adding a function of respiratory gated irradiation. This does not mean that disposition of the X-ray generation unit 20 and the detection unit 21 in which the effective field of view is 80% or more is essential.

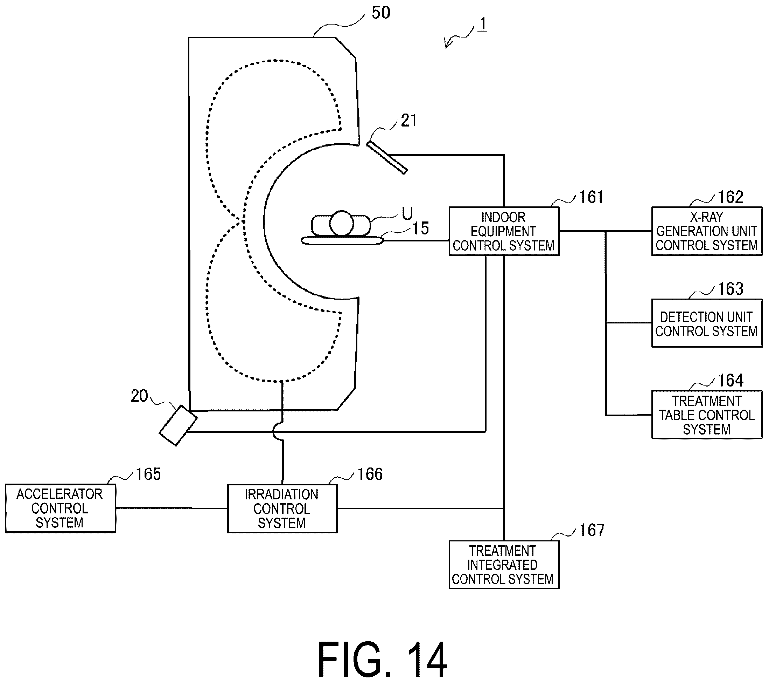

FIG. 14 is a system configuration diagram for carrying out the present example. A treatment integrated control system 167 is an upper system, and there is an irradiation control system 166 for performing treatment irradiation, and an indoor equipment control system 161 for performing patient positioning. The treatment integrated control system 167 instructs a converging electromagnet or an irradiation nozzle to enable the treatment from a desired angle. The converging electromagnet is excited so that irradiation can be performed from the desired angle, and the irradiation nozzle is driven to irradiate the isocenter O with the charged particle beam. The indoor equipment control system 161 drives the treatment table 15 using the treatment table control system 164, and transports the patient to a position for treatment irradiation. Then, an X-ray generation unit control system 162 performs X-ray exposure, and a detection unit control system 163 acquires an image.

According to the present invention, since the X-ray generation unit 20 or the detection unit 21 is not installed in the traveling direction of the charged particle beam emitted from the irradiation nozzle, there is no need to retract the X-ray generation unit 20 and the detection unit 21 at the time of treatment irradiation. As a result, since the installation accuracy of the X-ray generation unit 20 and the detection unit 21 does not change due to the drive for retraction, the treatment accuracy does not decrease compared to a case in which the X-ray generation unit 20 and the detection unit 21 are retracted to be stored in the ceiling or under the floor. Further, a space from the X-ray generation unit 20 to the detection unit 21 is not blocked due to the drive of the irradiation nozzle. Therefore, patient positioning can be performed while changing the treatment irradiation angle, and the treatment time can be shortened. As a result, the number of treated patients per treatment room can be increased.

Generally, in order to make it easier for a radiographer to grasp a patient's posture at a three-dimensional position when positioning the patient, the virtual plane 17 formed by the irradiation axis of the charged particle beam and a plane formed by an axis connecting the two pairs of X-ray generation units 20 and detection units 21 are perpendicular to each other and installed on horizontal and vertical beam lines on the same plane. When there are images captured from at least two directions, positioning is possible regardless of a disposition direction, and thus, the disposition of the X-ray generation unit 20 and the detection unit 21 of the present invention can achieve the same patient positioning accuracy as the related art.

The present invention contributes to improving the treatment accuracy for non-coplanar irradiation in which the treatment beam is radiated not only from a cross section perpendicular to a longitudinal axis direction of the patient but also from a non-coplanar plane, and the dose to normal tissue or major adjacent important organs is reduced. After the patient is positioned, treatment dependent on the accuracy of the movement of the treatment table in which the treatment table is rotated around the isocenter to start the irradiation so that irradiation is performed on a desired non-coplanar plane is performed. In the present invention, since the two pairs of X-ray generation units 20 and detection units 21 are disposed in the longitudinal axis direction of the patient, a large space is secured around the patient. Therefore, a patient's position can be confirmed with a constant X-ray imaging system while securing a space in which the treatment table rotates around the isocenter, improving the treatment accuracy in non-coplanar irradiation.

In the rotary gantry, since the two pairs of X-ray generation units 20 and detection units 21 rotate, patient positioning disposition is a disposition dependent on a forward direction, and in-body monitoring during irradiation is a disposition dependent on the irradiation angle. In the present example, in the respiratory gated irradiation, images obtained as in-body monitoring during patient positioning and irradiation may be evaluated in the same geometric disposition regardless of the irradiation angle during a series of treatments. When the patient positioning is performed, bones less affected by respiratory movement are used as landmarks. Next, in the respiratory gated irradiation, positioning considering a relative positional relationship between the movement of organs moving due to breathing and the bones is required, and two-stage position confirmation is performed. Similar confirmation is also performed for irradiation when a movement of an irradiation target changes over time due to movement within the body during irradiation such as a prostate affected by intestinal gas movement. In the present invention, since geometric dispositions of the two pairs of X-ray generation units 20 and detection units 21 at the time of patient positioning and time of in-body monitoring are the same, irradiation accuracy is improved in treatments that require organ position reproducibility, such as the respiratory gated irradiation.

In the present example, the two pairs of X-ray generation units 20 and detection units 21 are installed in a patient longitudinal direction relative to a non-rotary gantry, which prevents equipment installation and building structure from becoming complicated, and allows easy maintenance and miniaturization of the entire apparatus. In the case of the same disposition in a half gantry, at least one X-ray generation unit 20 and at least one detection unit 21 of the two pairs of X-ray generation units 20 and detection units 21 disposed on the upstream side of the beam is disposed inside a cylindrical rotary gantry. Therefore, a complex support is required for equipment installation inside the rotary gantry, and maintenance work is performed inside the rotary gantry, which places a heavy burden on a worker. Further, since the irradiation apparatus does not rotate, a building to house the irradiation apparatus has about half the size of a rotary gantry that requires a huge cylindrical structure with a height and depth of about 10 m as a rotation space, and installation cost of the treatment apparatus can be greatly reduced.

Example 3

Configuration of information processing device

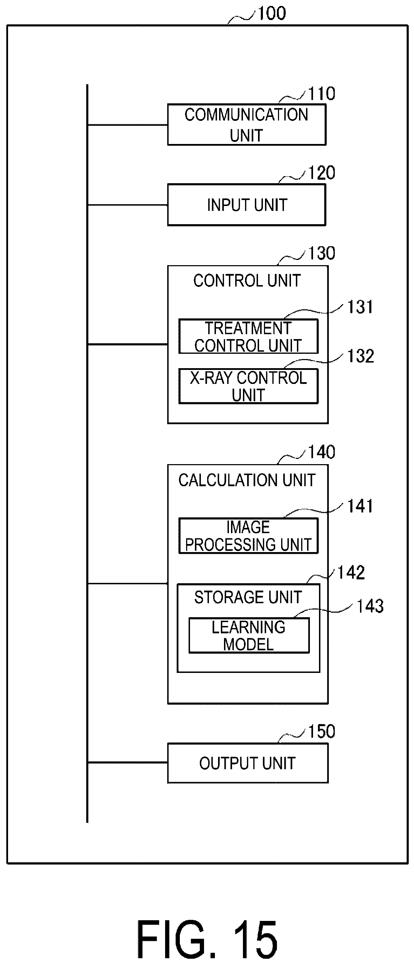

FIG. 15 is a block diagram illustrating an example of a configuration of the information processing device 100 that controls the charged particle beam irradiation system. The information processing device 100 is a computer system that controls the radiation of the charged particle beam from the charged particle beam irradiation apparatus to a treatment site of a patient, and may be realized by a so-called server device, a PC, a tablet terminal, or the like, but is not limited thereto. As illustrated in FIG. 15, the information processing device 100 includes a communication unit 110, an input unit 120, a control unit 130, and a calculation unit 140. The information processing device 100 may also include an output unit 150.

The communication unit 110 is a communication interface capable of communicating with an external apparatus. The communication unit 110 transmits instruction information for indicating timing of radiating the charged particle beam to an irradiation unit of the charged particle beam irradiation apparatus, for example, according to an instruction from the control unit 130. Further, the communication unit 110 instructs the X-ray generation unit 20 to radiate the X-rays and instructs the detection unit 21 to transmit the X-ray image according to an instruction from the control unit 130. Further, the communication unit 110 receives information related to the patient's treatment from an external apparatus and transmits the information to the control unit 130.

The input unit 120 is an input interface having a function of receiving input from an operator of the information processing device 100 and transmitting the input to the control unit 130. The input unit 120 may be realized by, for example, an input device such as a keyboard or a mouse, but is not limited thereto. The input unit 120 receives, for example, input of information related to the patient's treatment and transmits received input content to the control unit 130.

The control unit 130 is a processor having a function of controlling each unit of the information processing device 100. The control unit 130 functions as the information processing device 100 by executing a program built into the calculation unit 140. The control unit 130 includes a treatment control unit 131 and an X-ray control unit 132 as functions to be performed as the information processing device 100. The treatment control unit 131 has a function of transmitting instruction information to the charged particle beam irradiation apparatus to instruct the charged particle beam irradiation apparatus to radiate the charged particle beam via the communication unit 110. The X-ray control unit 132 instructs the X-ray generation unit 20 to radiate X-rays via the communication unit 110 and acquires the X-ray image detected by the detection unit 21. The X-ray control unit 132 transmits the acquired X-ray image to the treatment control unit 131.

The calculation unit 140 has a function of analyzing the X-ray image acquired by the X-ray control unit 132 and specifying the relative position of the target site as the treatment target with respect to the patient. An image processing unit 141 processes the X-ray image acquired by the X-ray control unit 132, analyzes a feature quantity in the image, and calculates a position of the treatment site of the target. In this case, an optimal combination of an existing image filter (for example, a noise removal filter or edge enhancement filter)) and the like is executed to reduce the burden on the radiographer at the time of patient positioning, but details thereof are omitted here. The calculation unit 140 also includes a memory unit 142. The memory unit 142 is a storage medium having a function of storing various programs and various types of data required for the operation of the information processing device 100. The memory unit 142 may be realized by, for example, a hard disk drive (HDD), a solid state drive (SSD), a flash memory, or the like, but is not limited thereto. The memory unit 142 stores a learning model 143 for determining timing of radiation of the charged particle beam. Also, as illustrated in FIG. 15, the position of the treatment site may be calculated via a learning model registered in the learning model 143. The learning model 143 is stored in the memory unit 142.

An example of the learning model 143 is a learning model that has learned a correspondence relationship between the X-ray image and a position of an organ, and specifies the relative position of the target site with respect to the patient using the X-ray image and information indicating the treatment site as input. The image processing unit 141 determines whether the treatment site of the target is located in an irradiation area including the irradiation position (isocenter) of the charged particle beam, and specifies the irradiation timing. Therefore, the learning model 143 is generated by learning a plurality of teaching data, which is information in which the X-ray image is associated with information indicating sites of various organs in the X-ray image. As an algorithm used for this learning model 143, for example, linear regression (+ regularization), support vector machine (+ kernel method), random forest, neural network, deep learning, k nearest neighbor method (kNN), or the like may be used in addition to known algorithms, but the present invention is not limited thereto. The learning model 143 is preferably a patient-specific model that has basically learned the X-ray image and a treatment target position of each patient who is a treatment target according to the patient, but may be a general-purpose model that has learned X-ray images and treatment target positions of a plurality of different patients. When a model specialized for each patient is prepared, it is expected that the accuracy of treatment can be improved compared to using the general-purpose model, while the general-purpose model does not require preparing a model each time a different patient is treated. Here, the X-ray image used for learning is an X-ray image captured by the X-ray generation unit 20 and the detection unit 21 disposed at the positions illustrated in FIGS. 7 to 9. That is, the X-ray image is an X-ray image obtained by radiating the X-rays in a human body height direction (longitudinal direction) and diagonally with respect to a horizontal direction relative to the human body height direction. Information (annotation) indicating which part of which organ is which part in the X-ray image is associated with the X-ray image to form teaching data, and to generate the learning model 143. In the present embodiment, it is preferable to prepare a first learning model 143 corresponding to the X-ray image captured by the detection unit 21a and a second learning model 143 corresponding to the X-ray image captured by the detection unit 21b. The annotation may be added by a medical professional or the like.

Further, an image used for learning here may also be a digital reconstructed radiograph generated by simulating an X-ray image from a CT image. Further, a plurality of learning models may be registered in the learning model 143. Another example of the learning model is a learning model that learns a correspondence relationship between, for example, an image filter and an irradiation site as a treatment target or exposure conditions in acquiring the X-ray image, and optimizes the image filter. An image filter selected by the learning model 143 is applied to the acquired X-ray image, and a target object is analytically detected by the image processing unit 141. In this case, the relative position of the target site with respect to the patient is also specified, and the image processing unit 141 can determine whether the treatment site of the target has arrived at the irradiation area including the irradiation position (isocenter) of the charged particle beam, and identify the relative position of the target site with respect to the patient for specifying the irradiation timing.

The output unit 150 has a function of outputting information designated by the control unit 130. The output unit 150 may be realized, for example, by a monitor or a speaker, but is not limited thereto. The output of information by the output unit 150 may be realized in the form of transmitting information to an external apparatus. The output unit 150 may output information about the treatment site under instructions from the control unit 130, for example.

Operation of the information processing device 100

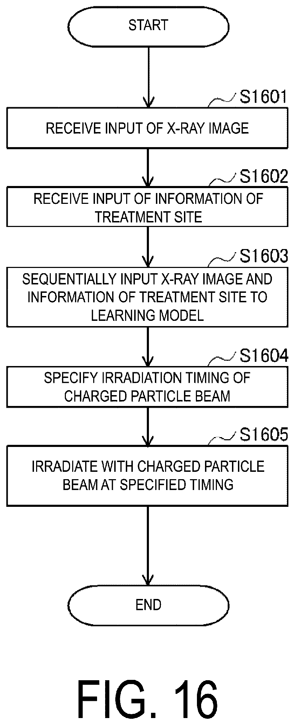

FIG. 16 is a flowchart illustrating an example of an operation of controlling the radiation of the charged particle beam of the information processing device 100.

As illustrated in FIG. 16, the communication unit 110 of the information processing device 100 receives an X-ray image. The communication unit 110 transmits the received X-ray image to the control unit 130. The control unit 130 receives input of an X-ray image for performing respiratory gating of proton beam treatment (step S1601). That is, the control unit 130 detects the X-rays radiated from the X-ray generation unit 20 using the detection unit 21, and receives input of the X-ray image obtained by the detection. As described above, the X-ray image is an image obtained by imaging the human body of a patient with X-rays irradiated to intersect with a virtual plane formed by a line through which the charged particle beam passes, rather than X-rays radiated in parallel to the charged particle beam (the same plane as a virtual plane formed by an irradiation path of the charged particle beam) as in the related art. In other words, the image is an image captured by the X-rays obliquely radiated to the human body. The X-ray image received here only needs to be information that allows the state of the in-body organ to be estimated, and may be a streaming video, may be a series of still images, or may be a moving image for each predetermined time unit (for example, 0.1 second units, but the present invention is not limited thereto).

Next, the communication unit 110 or the input unit 120 of the information processing device 100 receives input of information about the treatment site of the patient and transmits the information to the control unit 130 (step S1602). The information about the treatment site only needs to be information that allows the information processing device 100 to specify at least a relative position of the target site that is a treatment target with respect to the patient.

The control unit 130 sequentially receives input of X-ray images from the communication unit 110, and inputs the X-ray images and the information about the treatment site to the learning model 143 (step S1603). Accordingly, the treatment control unit 131 specifies timing at which the charged particle beam is radiated (step S1604).

The treatment control unit 131 transmits instruction information to the charged particle beam irradiation apparatus via the communication unit 110 to instruct the charged particle beam irradiation apparatus to perform irradiation so that the charged particle beam is radiated at specific timing (step S1605).

This allows the information processing device 100 to control the radiation of the charged particle beam at the appropriate timing.

In the present example, a relative position of the irradiation target organ with respect to an organ moving due to breathing and a positional relationship between another organ and the irradiation target organ are specified in the information processing device 100, thereby making it possible to reduce work of the radiographer and reduce radiation dose due to the X-ray exposure. For example, X-ray images for one breathing cycle are continuously acquired as internal body information, and the information is simultaneously acquired by a plurality of extracorporeal information acquisition devices installed outside. For example, by synchronizing a respiratory waveform or a movement of a body surface with in-body monitoring information, the information processing device 100 can predict the relative position of the irradiation target organ or a positional relationship between another organ and the irradiation target organ using only the extracorporeal information during treatment, and can perform irradiation at the appropriate timing. This eliminates the need for exposure during irradiation, and reduces an amount of radiation exposure to the patient. Further, it is possible to perform X-ray imaging and confirm the relative relationship only when there is concern about an unstable respiratory waveform based on the body outside monitoring information.

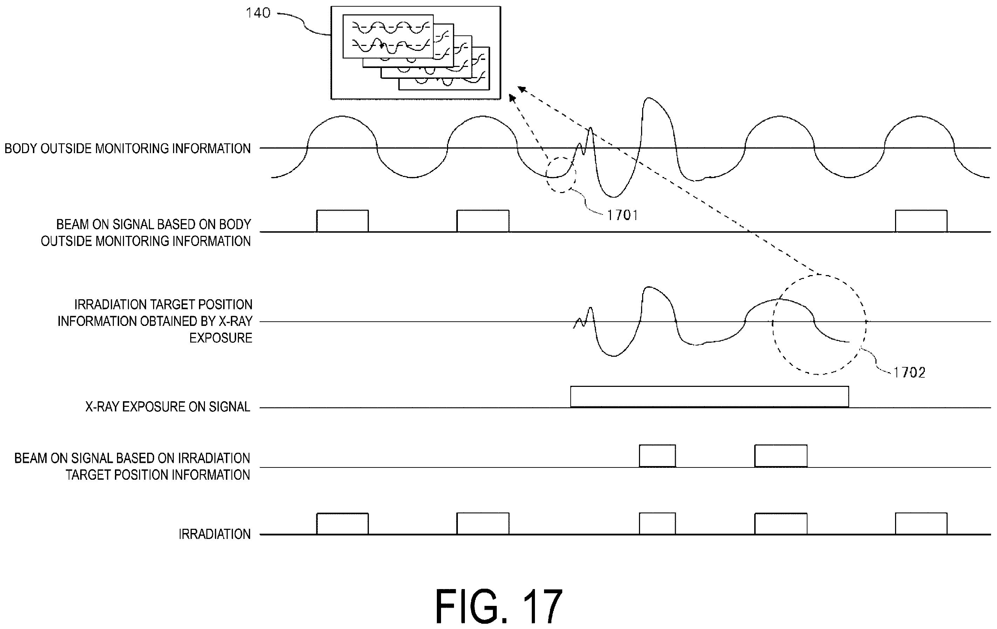

FIG. 17 illustrates a first control example in which an unstable respiratory waveform is predicted from data stored in the memory unit 142 of the calculation unit 140 and irradiation is performed, and is a timing chart schematically illustrating timing of X-ray irradiation, charged particle beam exposure, and the like. FIG. 17 illustrates from the top a waveform of the body outside monitoring information, a waveform of the beam ON signal based on the body outside monitoring information, a waveform of irradiation target position information obtained by the X-ray exposure, a waveform of an X-ray exposure ON signal, a waveform of the beam ON signal based on the irradiation target position information, and a radiation waveform of the charged particle beam. The body outside monitoring information may be information of a video obtained by filming the outside of the patient, may be, for example, a video obtained by filming a patient's abdomen, and may be waveform information schematically indicating a degree of abdominal expansion based on these videos. The beam ON signal based on the body outside monitoring information is information indicating an irradiation instruction for the charged particle beam, and is information about an irradiation instruction that is issued when the patient's breathing is stable based on the body outside monitoring information. The irradiation target position information obtained by the X-ray exposure is information indicating the position of the treatment site (irradiation target part of the charged particle beam) by imaging the inside of the patient's body by performing X-ray exposure. Further, the X-ray exposure ON signal is a signal for instructing the X-ray generation unit 20 to perform the X-ray exposure. Further, the beam ON signal based on the irradiation target position information is a signal for issuing instruction about the radiation of the charged particle beam to the treatment target site identified by X-ray exposure. The irradiation at a bottom of FIG. 17 indicates irradiation timing of the charged particle beam by the beam ON signal based on the irradiation target position information.

First, the image processing unit 141 analyzes the body outside monitoring information of the patient during treatment. The body outside monitoring information may be a captured video of the patient's abdomen, or the like. Basically, when the charged particle beam irradiation apparatus can detect that breathing is stable based on information from the body outside monitoring information (a location where the body outside monitoring information in FIG. 17 illustrates a stable wave system), the beam-ON signal is turned on based on the body outside monitoring information at the timing when the patient's body is in a predetermined state, for example, when a value of the body outside monitoring information in the example of FIG. 17 is a positive value (a predetermined value or more), that is, at timing when a patient's peritoneum expands to a predetermined degree or more, and radiation of the charged particle beam is executed.

At that time, the body outside monitoring information of the patient registered in the memory unit 142 of the calculation unit 140 and the data of the respiratory waveform at that time are referenced, and an unstable respiratory waveform is predicted in advance. For example, an unstable respiratory waveform 1701 surrounded by a dashed line on the respiratory waveform with an arrow part in the body outside monitoring information in FIG. 17 as a starting point is predicted. That is, the body outside monitoring information is input to the learning model 143 as an input, and it is estimated whether or not the patient's breathing is likely to become disturbed. When an unstable respiratory waveform is generated, it becomes impossible to perform treatment irradiation with high accuracy. Therefore, when the generation of an unstable respiratory waveform is predicted, imaging using X-rays is performed. That is, the X-ray generation unit 20 is instructed to start X-ray exposure and the detection unit 21 is instructed to perform imaging. When the treatment site (irradiation target position information) detected by the X-ray exposure reaches the irradiation position, the radiation of the charged particle beam is performed. While X-ray exposure is being executed, prediction of the respiratory waveform is continued using the learning model 143 and the body outside monitoring information. In a stage in which it is predicted from the body outside monitoring information that the respiratory waveform will be stable (a stage of an area surrounded by a dotted line 1702 in FIG. 17), X-ray imaging is stopped. This makes it possible to reduce unnecessary radiation dose. The process then returns to the control of the radiation of the charged particle beam based on the body outside monitoring information. Timing for returning to the control of the radiation of the charged particle beam based on the body outside monitoring information may be timing in a stage in which it is predicted that the respiratory waveform will be stable and a timing when the position of the treatment target site based on the body outside monitoring information is synchronized with the position of the treatment target site detected by the X-ray exposure. According to the first control example, since the X-ray exposure is performed only at timing when breathing is unstable, a processing load of the apparatus can be reduced compared to continuous X-ray exposure during treatment. Also, according to the first control example, even when detection of the treatment target site based on the body outside monitoring information becomes impossible due to disturbance in breathing, the detection of the treatment target site based on the body outside monitoring information can be resumed.

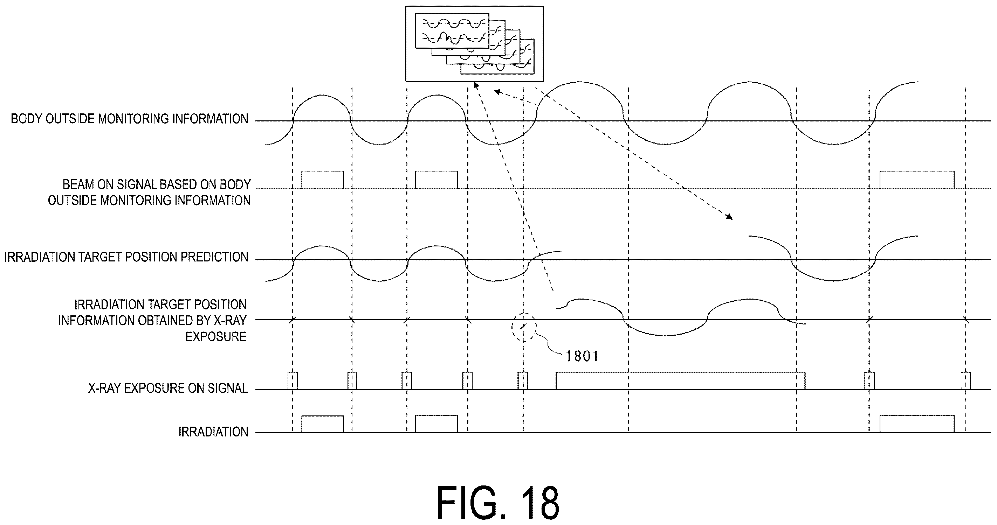

Alternatively, in the respiratory gated irradiation, the radiation dose due to X-ray imaging can be reduced by performing X-ray imaging only at start timing and end timing of an irradiation gate (timing near the start and end of an ON signal of a respiratory gate), confirming that the irradiation target is within a predetermined range, and correcting the timing of irradiation based on the respiratory waveform when there is a deviation from the initial relative relationship. FIG. 18 illustrates a second control example in which X-ray imaging is performed at timing near the ON signal and near the end of the ON signal of the respiratory gate that performs irradiation based on the body outside monitoring information. The content of each signal in FIG. 18 is the same as that in FIG. 17, but a waveform of irradiation target position prediction indicating the position of the treatment target site predicted from the body outside monitoring information is added. In the second control example, the detection of the irradiation target position by the X-ray exposure is periodically executed, as illustrated in the figure. Since the patient's breathing is basically cyclical, X-ray exposure is performed only at timing of inhalation and exhalation and it is determined whether the irradiation target position is a desired position. When unstable breathing is not predicted, ON control for the charged particle beam is executed based on the body outside monitoring information as in the first control example. That is, the radiation of the charged particle beam is executed at timing when the body outside monitoring information is equal to or greater than a predetermined value.

On the other hand, when it is detected based on the X-ray image that the irradiation target position is not the desired position (see dotted line 1801 in FIG. 18), it can be detected that there is a deviation in the relative relationship between the body outside monitoring information and periodic timing of X-ray exposure. When such a deviation is detected, the charged particle beam irradiation system switches the X-ray exposure from intermittent exposure to continuous exposure. The position of the treatment target site is continuously identified based on the X-ray image while the continuous X-ray exposure is performed. The radiation of the charged particle beam is executed at timing when the treatment target site reaches the irradiation position. When the patient's breathing becomes stable, the charged particle beam irradiation system synchronizes the position of the treatment target site specified by the X-ray exposure with the position of the treatment target site specified from the body outside monitoring information, and determines irradiation timing of the periodic X-ray exposure. When the irradiation timing is determined, the continuous X-ray exposure is stopped and intermittent X-ray exposure is resumed.

Further, in the case of a combination of the irradiation beam apparatus 50 having a non-rotary gantry of Example 2 and Example 3, since a large space can be secured around the patient, an extracorporeal information acquisition device such as a 3D camera or an ultrasound device can be installed. A space can be secured to install a radiation-free apparatus such as an MRI that is a relatively large apparatus in order to specify the position more precisely, in addition to the extracorporeal information. For example, although the MRI has a lower frame rate than a frame rate of an X-ray image, the relative position of the irradiation target organ or a positional relationship between the irradiation target organ and the other organ can be specified with high accuracy from the information processing device 100, and irradiation can be performed at appropriate timing.

FIG. 19 is a diagram schematically illustrating a relative positional relationship between the X-ray generation unit 20 and the detection unit 21 in a full gantry and half gantry of the related art, and a relative positional relationship between the X-ray generation unit and the detection unit in the present invention. FIG. 19(a) is a diagram illustrating an example of a disposition of the X-ray generation unit and the detection unit in the charged particle beam irradiation system according to the present invention, which is not a rotary gantry, FIG. 19(b) is a diagram illustrating a relative positional relationship between an X-ray generation unit and a detection unit in a full gantry, and FIG. 19(c) is a diagram illustrating a relative positional relationship between an X-ray generation unit and a detection unit in a half gantry. Dotted lines in FIGS. 19(a) to 19(c) indicate the virtual plane P (17) generated from a plurality of trajectories of the radiated charged particle beam. F1 and F2 indicate the respective detection units, and X1 and X2 indicate the X-ray generation units. Further, for upstream and downstream, the upstream side is a side where the charged particle beam is generated in the charged particle beam irradiation apparatus, and the downstream side is a side where the charged particle beam is emitted.

As is clear from a comparison of FIGS. 19(a), 19(b) and 19(c), the charged particle beam irradiation system according to the present invention, which is not a rotary gantry, and the full gantry or the half gantry of the related art are the same in that axes connecting the pairs of X-ray generation units (X1 and X2) and detection units (F1 and F2), that is, a line connecting the X-ray generation unit X1 to the detection unit F1 and a line connecting the X-ray generation unit X2 to the detection unit F2 (both not illustrated) intersect on the virtual plane P. On the other hand, the charged particle beam irradiation system according to the present invention, which is not a rotary gantry, and the full gantry or the half gantry of the related art can be said to differ in that a direction from the upstream to the downstream of the apparatus is parallel or perpendicular to the virtual plane P. Also, as is clear from a comparison of FIGS. 19(a) and 19(c), the charged particle beam irradiation system according to the present invention, which is not a rotary gantry, and the full gantry of the related art differ in whether or not all the detection units are located upstream of the X-ray generation units. Further, as is clear from a comparison of FIGS. 19(a) and 19(b), the charged particle beam irradiation system according to the present invention and the full gantry of the related art are different in whether or not the X-ray generation units (X1 and X2) and the detection units (F1 and F2) are on the same side of the virtual plane P. From the above, one of characteristics of the charged particle beam irradiation system according to the present invention, which is not a rotary gantry, is that two conditions including (i) the X-ray generation unit and the detection unit are not on the same side with respect to the virtual plane P, and (ii) all detection units (or X-ray generation units) are disposed upstream of the X-ray generation unit (or detection unit) are satisfied.

Conclusion

In the charged particle beam irradiation system according to the embodiment, a rotary gantry is not used as an apparatus for radiating a charged particle beam, the first X-ray generation unit and the second X-ray generation unit are disposed symmetrically with respect to the virtual plane formed by the plurality of trajectories of the charged particle beam selectable by the charged particle beam irradiation apparatus, and when the side where the charged particle beam is incident on the charged particle beam irradiation apparatus is defined as the upstream side and the side where the charged particle beam is emitted from the charged particle beam irradiation apparatus is defined as the downstream side, the first detection unit and the second detection unit are positioned upstream or downstream of the first X-ray generation unit and the second X-ray generation unit, thereby making it possible to realize respiratory gating method using X-rays while curbing an increase in size of the charged particle beam irradiation apparatus.

The X-ray generation unit and the detection unit are disposed on the ground and the ceiling on the left and right sides of the charged particle beam irradiation apparatus, the X-ray generation unit and the detection unit are separated from the irradiation apparatus, and thus, a large space is secured around the patient without the need to retract the X-ray generation unit and the detection unit when the treatment beam is radiated. Therefore, the installation accuracy of the X-ray generation unit and the detection unit does not change due to the drive caused by insertion/retraction, and the treatment accuracy does not decrease. Further, it is possible to perform patient positioning while changing the treatment irradiation angle, to shorten the treatment time, and to increase the number of treated patients per treatment room. Further, a patient's position can be confirmed in a certain geometric disposition for X-ray imaging while securing the space in which the treatment table rotates around the isocenter, and the treatment accuracy in non-coplanar irradiation is improved.

In the respiratory gated irradiation, during a series of treatments, images acquired for the patient positioning and in-body monitoring during the irradiation may be evaluated in the same geometric disposition regardless of the irradiation angle. When patient positioning is performed, bones less affected by respiratory movement are used as a reference, positioning considering a relative positional relationship between the movement of organs moving due to breathing and the bones is required, and two-stage position confirmation is performed. In this case, since the geometric disposition of the two pairs of X-ray generation units and detection units at the time of patient positioning and the time of in-body monitoring is the same, the treatment accuracy of respiratory gated irradiation is improved.

Modification Examples

The charged particle beam irradiation system and the information processing device 100 described in the embodiment are not limited to the aspects described in the embodiment. Appropriate modifications are possible within the knowledge of those skilled in the art. Hereinafter, various modification examples will be described.

(1) The information processing device 100 may include a learning unit for learning the learning model 143. That is, the information processing device 100 may include a function of generating the learning model 143 by performing learning according to a predetermined algorithm based on a plurality of pieces of teaching data.
Further, the information processing device 100 may include a re-learning unit that receives input of new teaching data and re-learns the learning model 143. With the re-learning unit, the accuracy of estimation can be improved by obtaining more teaching data (knowledge).

(2) In the embodiment, a case where there is one learning model 143 has been described, but there may be a plurality of learning models 143. That is, the learning model 143 may be created for each organ that is a treatment target, for example. It is possible to perform more accurate learning, and to realize highly accurate charged particle beam irradiation by subdividing the learning model 143 for each organ.

(3) In the embodiment, the learning model 143 may be created for each patient who receives treatment, and in this case, the learning model 143 may perform learning by assigning a patient's treatment site as annotation information to the X-ray image. More accurate charged particle beam irradiation can be realized by generating the learning model 143 for each patient. Further, the learning model 143 may be not only constructed from information such as information of an image captured before the treatment of the patient who receives treatment, but also constructed before the treatment of the patient from patient data such as X-ray images of unspecified patients who have been treated. Accordingly, it is expected that the accuracy of treatment is improved by increasing the number of learning pieces of data. This approach is expected to improve of the positioning accuracy of skeletal positioning using X-ray images of bones, as well as the irradiation control at the time of treatment of organs moving due to respiratory movement.

(4) In the embodiment, an angle between the X-ray and the virtual plane 17 is arbitrary, and the X-ray generation unit 20 only needs to be configured to be included on both sides of the charged particle beam irradiation apparatus not to interfere with the movement of the movable vehicle 10. In this case, the X-ray generation unit 20 is provided as near the charged particle beam irradiation apparatus as possible to prevent the treatment room from becoming large, but the X-ray generation unit 20 is preferably disposed in a position where a housing of the charged particle beam irradiation apparatus or the movable vehicle 10 at the treatment position do not interfere with the acquisition of X-ray images. Further, in the embodiment, an example in which the two X-ray generation units 20 and the corresponding detection units 21 are provided has been described, but these may be a single pair, or more than two pairs of X-ray generation units and corresponding detection units may be provided.

(5) The program of each embodiment of the present disclosure may be provided in a state in which the program is stored in a storage medium readable by the information processing device. The storage medium may be a "non-transitory tangible medium" capable of storing the program. The program may include, for example, a software program or an information processing device program.

The storage medium may, where appropriate, include one or more semiconductor-based or other integrated circuits (ICs) (for example, field programmable gate arrays (FPGAs) or application specific ICs (ASICs)), hard disk drives (HDDs), hybrid hard drives (HHDs), optical discs, optical disk drives (ODDs), magneto-optical disks, magneto-optical drives, floppy diskettes, floppy disk drives (FDDs), magnetic tapes, solid state drives (SSDs), RAM drives, secure digital cards or drives, any other suitable storage media, or any suitable combination of two or more of these. The storage medium may, where appropriate, be volatile, non-volatile, or a combination of volatile and non-volatile.

The program of the present disclosure may also be provided to the information processing device 100 via any transmission medium (such as a communication network or broadcast waves) capable of transmitting the program.

Further, each embodiment of the present disclosure can be realized in the form of a data signal embedded in carrier waves, in which the program is embodied by electronic transmission.

The program of the present disclosure may be implemented using any programming language, such as a scripting language such as JavaScript (trade name) or Python, C language, Go language, Swift, Koltin, and Java (trade name).

(6) The present invention may also be a method for controlling the charged particle beam from the charged particle beam irradiation apparatus in the information processing device 100. That is, an aspect of the present invention provides a method for controlling radiation of a charged particle beam in a charged particle beam irradiation system including a charged particle beam irradiation apparatus including a deflection electromagnet that deflects a charged particle beam to continuously change the irradiation angle of the charged particle beam to an isocenter, and an irradiation nozzle that moves continuously along a shape on an emission side of an effective magnetic field region of the deflection electromagnet, an X-ray generation unit that radiates X-rays, and a detection unit that detects the X-rays, wherein the X-ray generation unit and the detection unit are disposed so that the charged particle beam emitted from the deflection electromagnet passes through the irradiation nozzle and radiated to the isocenter, and a virtual line connecting the X-ray generation unit to the detection unit intersects with, but is not parallel to, a virtual plane formed by the charged particle beam radiated from the irradiation nozzle to the isocenter, wherein an information processing device executes a first reception step of receiving an X-ray image detected by the detection unit, a second reception step of receiving information about a treatment site of a patient receiving treatment with the charged particle beam, and a control step of controlling timing of radiation of the charged particle beam using a learning model that has learned a relationship between the X-ray image detected by the detection unit and a position of an organ in the X-ray image, the X-ray image received in the first reception step, and information about the treatment site of the patient received in the second reception step.

(7) Processing of the flowchart illustrated in FIG. 16 can be appropriately changed within a range in which similar results can be obtained. For example, for the processing of step S1601 and step S1602, the processing of step S1602 may be first executed, or the processing of step S1601 and step S1602 may be executed simultaneously.

Reference Signs List

1 Charged particle beam irradiation system
10 Movable vehicle
11 Irradiation nozzle
15 Treatment table
16 Arm
20, 20a, 20b X-ray generation unit
21, 21a, 21b Detection unit
50 Charged particle beam irradiation apparatus
70 Distribution electromagnet
80 Deflection electromagnet
100 Information processing device
110 Communication unit
120 Input unit
130 Control unit
131 Treatment control unit
132 X-ray control unit
140 Calculation unit
141 Image processing unit
142 Storage unit
143 Learning model
150 Output unit

**Claims**

1. A charged particle beam irradiation system comprising:

   a charged particle beam irradiation apparatus on which a charged particle beam transported after being emitted from an accelerator is incident and from which the charged particle beam is emitted toward an isocenter; and
   a first X-ray generation unit and a first detection unit, and a second X-ray generation unit and a second detection unit, wherein
   respective X-rays generated from the first X-ray generation unit and the second X-ray generation unit pass through the isocenter and are detected by the first detection unit and the second detection unit,
   the first X-ray generation unit and the second X-ray generation unit are disposed to sandwich a virtual plane between the first X-ray generation unit and the second X-ray generation unit, the virtual plane being formed by a plurality of trajectories of the charged particle beam selectable by the charged particle beam irradiation apparatus, and
   when a side where the charged particle beam is incident on the charged particle beam irradiation apparatus is defined as an upstream side and a side where the charged particle beam is emitted from the charged particle beam irradiation apparatus is defined as a downstream side, the first detection unit and the second detection unit are located upstream or downstream of the first X-ray generation unit and the second X-ray generation unit.

2. The charged particle beam irradiation system according to claim 1, wherein the plurality of trajectories are radiated from an irradiation unit fixed and installed with a certain interval in the charged particle beam irradiation apparatus.

3. The charged particle beam irradiation system according to claim 1, wherein the first X-ray generation unit and the second X-ray generation unit are disposed symmetrically with respect to the virtual plane.

4. The charged particle beam irradiation system according to claim 1, wherein

   the plurality of trajectories are formed under an influence of a converging electromagnet including a pair of coils disposed to sandwich a path of the charged particle beam between the pair of coils,
   the converging electromagnet is configured to generate an effective magnetic field region in which a magnetic field is oriented in a direction perpendicular to a traveling direction of the charged particle beam when a current is input to the pair of coils, the traveling direction of the charged particle beam being an X-axis, the direction perpendicular to the X-axis being a Z-axis, an axis perpendicular to both the X-axis and the Z-axis being a Y-axis,
   the charged particle beam deflected at a deflection angle $\varphi$ with respect to the X-axis at a deflection starting point Q and incident on the effective magnetic field region is deflected by the effective magnetic field region and radiated to the isocenter at an irradiation angle $\theta$ with respect to the X-axis on an XY plane,
   any point P2 on a boundary of the effective magnetic field region on an emission side of the charged particle beam are located at an equal distance r1 from the isocenter,
   a point P1 on a boundary of the effective magnetic field region on an incidence side of the charged particle beam and the any point P2 are on an arc with a radius r2 and a central angle $(\theta+\varphi)$, and
   a distance R between the deflection starting point Q and the point P1 satisfies a relational equation (4):
   [Math. 1]

$$R = \sqrt{L^2 + r_1^2 - 2L(r_1 \cos\theta + r_2 \sin\theta)} \quad (4)$$

   where L is a distance between the deflection starting point Q and the isocenter.

FIG. 1

(a)

(b)

FIG. 2

EP 4 570 311 A1

FIG. 3

FIG. 4

EP 4 570 311 A1

FIG. 5

(a)

1

(b)

1

FIG. 6

FIG. 7

FIG. 8

EP 4 570 311 A1

FIG. 9

FIG. 10

FIG. 11

(a)

(b)

50

11

21

X-RAYS

U
15

16'

α

20

50

21(21b)

21(21a)

11

U
15

16'

10

β

17

20(20a)

20(20b)

EP 4 570 311 A1

# FIG. 12

(a)

(b)

FIG. 13

FIG. 14

100

COMMUNICATION
UNIT
110

INPUT UNIT
120

CONTROL UNIT
130

TREATMENT
CONTROL UNIT
131

X-RAY CONTROL
UNIT
132

CALCULATION UNIT
140

IMAGE
PROCESSING UNIT
141

STORAGE UNIT
142

LEARNING
MODEL
143

OUTPUT UNIT
150

FIG. 15

START

↓ S1601

RECEIVE INPUT OF X-RAY IMAGE

↓ S1602

RECEIVE INPUT OF INFORMATION OF
TREATMENT SITE

↓ S1603

SEQUENTIALLY INPUT X-RAY IMAGE AND
INFORMATION OF TREATMENT SITE TO
LEARNING MODEL

↓ S1604

SPECIFY IRRADIATION TIMING OF
CHARGED PARTICLE BEAM

↓ S1605

IRRADIATE WITH CHARGED PARTICLE
BEAM AT SPECIFIED TIMING

↓

END

# FIG. 16

EP 4 570 311 A1

140

BODY OUTSIDE MONITORING INFORMATION

~1701

BEAM ON SIGNAL BASED ON BODY
OUTSIDE MONITORING INFORMATION

IRRADIATION TARGET POSITION
INFORMATION OBTAINED BY X-RAY
EXPOSURE

~1702

X-RAY EXPOSURE ON SIGNAL

BEAM ON SIGNAL BASED ON IRRADIATION
TARGET POSITION INFORMATION

IRRADIATION

FIG. 17

FIG. 18

EP 4 570 311 A1

(a)

UPSTREAM

F1      F2

P

X2      X1

DOWNSTREAM

(b)

X1      F2

UPSTREAM        DOWNSTREAM

X2      F1

P

(c)

F1      F2

UPSTREAM        DOWNSTREAM

X2      X1

P

# FIG. 19

## INTERNATIONAL SEARCH REPORT

| International application No. |
| --- |
| **PCT/JP2023/020710** |

### A. CLASSIFICATION OF SUBJECT MATTER

*A61N 5/10*(2006.01)i
FI:  A61N5/10 M; A61N5/10 H

According to International Patent Classification (IPC) or to both national classification and IPC

### B. FIELDS SEARCHED

Minimum documentation searched (classification system followed by classification symbols)

A61N5/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2007-37629 A (HITACHI, LTD.) 15 February 2007 (2007-02-15) paragraphs [0018]-[0022], [0033], fig. 1, 2 | 1, 3 |
| Y | | 2, 4 |
| X | CN 105920745 A (SICHUAN UNIVERSITY) 07 September 2016 (2016-09-07) paragraphs [0003], [0017]-[0019], [0028], [0032], fig. 1, 2 | 1, 3 |
| Y | | 2, 4 |
| Y | JP 2019-166098 A (HITACHI, LTD.) 03 October 2019 (2019-10-03) paragraph [0042], fig. 1, 2 | 2 |
| Y | JP 2019-180654 A (B DOT MEDICAL INC.) 24 October 2019 (2019-10-24) paragraphs [0008], [0050]-[0052], fig. 9 | 4 |
| A | JP 2003-220151 A (MITSUBISHI HEAVY INDUSTRIES, LTD.) 05 August 2003 (2003-08-05) paragraphs [0021]-[0025], fig. 1 | 1 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 July 2023** | **18 July 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/020710**

| Patent document cited in search report | | | Publication date (day/month/year) | Patent family member(s) | | | Publication date (day/month/year) |
|---|---|---|---|---|---|---|---|
| JP | 2007-37629 | A | 15 February 2007 | (Family: none) | | | |
| CN | 105920745 | A | 07 September 2016 | (Family: none) | | | |
| JP | 2019-166098 | A | 03 October 2019 | (Family: none) | | | |
| JP | 2019-180654 | A | 24 October 2019 | US | 2019/0311878 | A1 | |
| | | | | paragraphs [0010]-[0016], [0131], [0132], fig. 9 | | | |
| | | | | US | 2019/0311879 | A1 | |
| | | | | EP | 3549637 | A1 | |
| | | | | EP | 3653263 | A1 | |
| | | | | KR | 10-1974425 | B1 | |
| | | | | CN | 110339491 | A | |
| | | | | JP | 6364141 | B1 | |
| JP | 2003-220151 | A | 05 August 2003 | (Family: none) | | | |

Form PCT/ISA/210 (patent family annex) (January 2015)

**REFERENCES CITED IN THE DESCRIPTION**

**Patent documents cited in the description**

- JP 6523076 B
- JP 6158334 B
- JP 6387476 B